(19) Europäisches Patentamt / European Patent Office / Office européen des brevets

(11) **EP 3 153 888 B1**

(12) **FASCICULE DE BREVET EUROPEEN**

(45) Date de publication et mention
de la délivrance du brevet:
**25.04.2018 Bulletin 2018/17**

(51) Int Cl.:
***G01T 1/29*** *(2006.01)*

(21) Numéro de dépôt: **16192679.5**

(22) Date de dépôt: **06.10.2016**

(54) **PROCÉDÉ DE CORRECTION D'UN SPECTRE**

VERFAHREN ZUR KORREKTUR EINES SPEKTRUMS

METHOD FOR CORRECTION OF A SPECTRUM

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **09.10.2015 FR 1559646**

(43) Date de publication de la demande:
**12.04.2017 Bulletin 2017/15**

(73) Titulaire: **Commissariat à l'Énergie Atomique
et aux Énergies Alternatives
75015 Paris (FR)**

(72) Inventeurs:
- **SOSSIN, Artur
  38000 GRENOBLE (FR)**
- **REBUFFEL, Veronique
  38700 CORENC (FR)**
- **TABARY, Joachim
  38100 GRENOBLE (FR)**

(74) Mandataire: **GIE Innovation Competence Group
310, avenue Berthelot
69372 Lyon Cedex 08 (FR)**

(56) Documents cités:
**US-A1- 2012 140 891**

- **KAI YANG ET AL: "A breast-specific,
  negligible-dose scatter correction technique for
  dedicated cone-beam breast CT: a physics-based
  approach to improve Hounsfield Unit accuracy",
  PHYSICS IN MEDICINE AND BIOLOGY,
  INSTITUTE OF PHYSICS PUBLISHING, BRISTOL
  GB, vol. 59, no. 21, 13 octobre 2014 (2014-10-13),
  pages 6487-6505, XP020272040, ISSN: 0031-9155,
  DOI: 10.1088/0031-9155/59/21/6487 [extrait le
  2014-10-13]**
- **SOSSIN A ET AL: "Fast scattering simulation tool
  for multi-energy x-ray imaging", NUCLEAR
  INSTRUMENTS & METHODS IN PHYSICS
  RESEARCH. SECTION A: ACCELERATORS,
  SPECTROMETERS, DETECTORS, AND
  ASSOCIATED EQUIPMENT, vol. 802, 9 septembre
  2015 (2015-09-09), pages 60-66, XP029287123,
  ISSN: 0168-9002, DOI:
  10.1016/J.NIMA.2015.08.073**

**Description**

## DOMAINE TECHNIQUE

Le domaine technique de l'invention est le traitement de spectres d'un rayonnement transmis par un objet, ce dernier étant irradié par une source de rayonnement ionisant, en particulier un rayonnement X. L'objectif de l'invention est d'obtenir une pluralité de spectres à l'aide d'un détecteur pixellisé, et d'effectuer une correction de tout ou partie des spectres, de façon à limiter l'influence d'une composante représentative d'un rayonnement diffusé. Les applications se trouvent dans l'imagerie médicale ou dans le contrôle non destructif.

## ART ANTERIEUR

Le contrôle d'objets par rayonnement X, dans le domaine médical ou industriel, est très répandu. Les procédés existants consistent à disposer un objet entre une source de rayonnement et un détecteur, puis à irradier l'objet à l'aide de la source. Le détecteur forme alors une image, généralement en deux dimensions, du rayonnement transmis par l'objet. Cette image est représentative de l'atténuation, par l'objet, du rayonnement émis par la source.

Le rayonnement transmis par l'objet comporte généralement une composante résultant de la diffusion, par l'objet, du rayonnement émis par la source. Elle est d'autant plus significative que l'énergie du rayonnement est faible et/ou que l'objet est constitué de matériaux dont le numéro atomique est élevé. Cette composante, appelée communément rayonnement de diffusion, perturbe l'interprétation des images, car elle n'est qu'indirectement liée à l'atténuation par l'objet. De plus, alors que le rayonnement non diffusé, dit rayonnement primaire, se propage entre la source et le détecteur selon une trajectoire rectiligne, le rayonnement diffusé a pour origine un point quelconque de l'objet, et sa trajectoire, depuis ce point origine, est distribuée selon différents angles. On recherche donc à estimer cette composante de diffusion, de façon à l'extraire du signal mesuré par le détecteur, préalablement au traitement des images en vue de leur interprétation.

Des outils de simulation numérique permettent de déterminer le rayonnement primaire et le rayonnement de diffusion ayant traversé un objet analysé. Un exemple en est donné dans la pu blication Sossin A. "Fast scattering simulation tool for multi-energy x-ray imaging" Nuclear Instruments and Methods in Physics Research, A 802 (2015) 60-66. De tels outils facilitent le développement de systèmes de radiographie ou de tomographie.

La demande de brevet US2012/0140891 décrit un procédé pour évaluer, à partir de mesures spectrométriques, une composante dite de diffusion primaire, correspondant à des photons n'ayant diffusé qu'une seule fois dans un échantillon analysé. Pour cela, une source de rayonnement X et un détecteur spectrométrique sont disposés face à un objet à analyser. Le détecteur spectrométrique détecte un rayonnement rétrodiffusé par l'objet, et en forme un spectre en énergie. Le procédé permet de déterminer un spectre de la composante de diffusion primaire.

Dans le domaine de l'imagerie médicale, de nombreuses méthodes ont été développées pour tenter d'estimer et de réduire la part du rayonnement diffusé, de façon à obtenir une image essentiellement représentative du rayonnement non diffusé, dit rayonnement primaire, se propageant entre la source et l'objet selon une direction rectiligne.

Par exemple, la publication Zhu L, « Scatter Correction Method for X-ray CT Using Primary Modulation : Theory and Preliminary Results », IEEE Transactions on Médical Imaging, Vol. 25, N° 12, dec 2006, décrit un procédé consistant à interposer un masque amovible, prenant la forme d'une grille, entre une source de rayons X et un objet. Cette publication est basée sur le fait qu'un tel masque engendre une modulation spatiale significative du rayonnement primaire, dans des fréquences spatiales élevées, tandis que son influence sur le rayonnement diffusé, dans les fréquences spatiales faibles, est moins importante. Ainsi, dans un espace fréquentiel, il est possible d'établir une discrimination entre le rayonnement primaire et le rayonnement diffusé.

Par ailleurs, la publication Ning R, « X-ray Scatter Correction Algorithm for Cone Beam CT-Imaging », Med.Phys. 31 (5), May 2004, décrit également un procédé consistant à interposer un masque amovible entre une source de rayons X et un objet. Le masque permet une atténuation telle que lorsqu'il est disposé entre la source et l'objet, le rayonnement mesuré par le détecteur est considéré comme représentatif du seul rayonnement diffusé. Autrement dit, ce masque permet une atténuation, considérée comme totale, du rayonnement primaire.

Le principe d'un masque amovible est également décrit dans la publication Yang K, "A breast-specific, negligible-dose scatter correction technique for dedicated cone-beam breast CT : a physics-based approach to improve Hounsfield Unit accuracy", Phys.Med.Biol. 59 (2014) 6487-6505.

Les détecteurs actuels permettent l'obtention d'images en deux dimensions dont la qualité ne cesse de s'améliorer. Une évolution récente est l'apparition de détecteurs permettant l'acquisition d'images spectrales, c'est-à-dire d'images selon différentes bandes d'énergie. Ces détecteurs, fréquemment basés sur des détecteurs semi-conducteurs ayant une fonction spectrométrique, ajoutent une dimension spectrale aux données acquises, ces dernières étant généralement obtenues selon deux dimensions. Il est alors possible d'obtenir une image du rayonnement transmis par l'objet selon différentes bandes d'énergie.

Or, les méthodes précitées ne concernent pas l'imagerie spectrale. Les inventeurs proposent une solution alternative, basée sur l'imagerie spectrale, permettant une correction efficace du rayonnement détecté par le détecteur, de façon à en extraire le rayonnement primaire, et cela simultanément dans plusieurs bandes d'énergie. Les essais présentés à la fin de la description témoignent de l'efficacité de la méthode.

## EXPOSE DE L'INVENTION

L'invention est un procédé de correction d'un spectre d'un rayonnement électromagnétique ionisant transmis par un objet,

- l'objet étant disposé entre une source d'irradiation et un détecteur, la source d'irradiation étant apte à émettre un rayonnement électromagnétique ionisant, dit rayonnement incident, vers ledit objet ;
- le détecteur (20) comportant des pixels, chaque pixel étant apte à détecter un rayonnement transmis par l'objet vers le détecteur, et à en acquérir un spectre, le rayonnement transmis comportant un rayonnement de diffusion, causé par la diffusion du rayonnement incident dans l'objet, et un rayonnement primaire,

le procédé comportant les étapes suivantes :

a) interposition d'un masque entre la source et l'objet, et acquisition, par plusieurs pixel, d'un premier spectre d'un premier rayonnement transmis par l'objet, ledit masque comportant des éléments absorbants, aptes à absorber une partie dudit rayonnement incident, et dont une projection sur le détecteur définit un premier groupe de pixels ;
b) obtention, pour chaque pixel du premier groupe de pixels, d'un deuxième spectre d'un deuxième rayonnement transmis par l'objet sur le détecteur, en l'absence dudit masque ;
c) comparaison, pour chaque pixel dudit premier groupe de pixels, du premier spectre et du deuxième spectre, de façon à obtenir un spectre de comparaison;
d) pour chaque pixel du premier groupe de pixels, application d'une matrice de passage, préalablement établie, audit spectre de comparaison, pour obtenir une estimation d'un spectre dit primaire, représentant le rayonnement primaire transmis par l'objet audit pixel, et estimation d'un spectre de diffusion, représentatif du rayonnement de diffusion transmis par l'objet ;
e) pour tout ou partie des pixels du détecteur, à partir de chaque spectre de diffusion estimé lors de l'étape d), correction du deuxième spectre ou du premier spectre, de façon à obtenir un spectre corrigé.

Pour chaque pixel du détecteur, le spectre corrigé constitue alors une estimation du spectre d'un rayonnement primaire transmis par l'objet. Il représente plus fidèlement les propriétés d'atténuation du rayonnement ionisant par l'objet.

L'étape e) peut comporter :

- préalablement à ladite correction, une estimation d'un spectre de diffusion pour l'ensemble des pixels du détecteur.
- pour chaque pixel, une soustraction dudit spectre de diffusion estimé audit deuxième spectre ou audit premier spectre.

L'étape e) peut comporter :

- pour chaque pixel du premier groupe, une correction du deuxième spectre, obtenu lors de l'étape b) en lui soustrayant le spectre de diffusion estimé lors de l'étape d) ;
- pour les pixels n'appartenant pas au premier groupe, une correction du deuxième spectre, obtenu lors de l'étape b) ou du premier spectre, obtenu lors de l'étape a) par uns soustraction du spectre de diffusion estimé lors de l'étape d).

Selon un mode de réalisation, lors de l'étape b), pour chaque pixel du premier groupe, le deuxième spectre est obtenu :

- soit par une acquisition dudit deuxième spectre par ledit pixel ;
- soit par une estimation sur la base d'au moins un premier spectre acquis par au moins un pixel n'appartenant pas audit premier groupe.

Selon un mode de réalisation :

- l'étape a) est mise en oeuvre dans une pluralité de configurations, à chaque configuration étant associée une position du détecteur et de la source par rapport à l'objet, de façon à obtenir, à chaque configuration, et pour chaque pixel, un premier spectre, à chaque configuration étant également associé un premier groupe de pixel ;

- l'étape b) comporte, pour tout ou partie des pixels du premier groupe associé à une configuration, une détermination d'un deuxième spectre à partir d'un premier spectre obtenu selon une autre configuration. Ainsi, pour certains pixels du détecteur, le premier spectre peut être obtenu selon une configuration, et le deuxième spectre peut être obtenu selon une autre configuration, cette dernière étant différente de celle selon laquelle ledit premier spectre est obtenu.

Cela évite d'utiliser un masque amovible. On tire alors profit que des pixels appartiennent à un premier groupe de pixel selon une configuration, et n'appartiennent pas au premier groupe de pixel associé à ladite autre configuration, et sont alors directement exposés à la source de rayonnement. Le rayonnement qu'ils reçoivent, selon cette autre configuration, est considéré comme représentatif du rayonnement qu'ils recevraient, dans ladite configuration, en l'absence du masque.

Selon ce mode de réalisation, à chaque configuration est associé un paramètre, qu'au moins un deuxième spectre selon une configuration associée à un premier paramètre est obtenu à partir d'un premier spectre obtenu selon une autre configuration associée à un deuxième paramètre, différent du premier paramètre. Ce paramètre peut être angle d'inclinaison de la source et/ou du détecteur par rapport à l'objet.

Selon un mode de réalisation, dit mode de réalisation tomographique, les étapes a) à e) sont mise en oeuvre selon une pluralité de configurations, à chaque configuration étant associée une position du détecteur et de la source par rapport à l'objet, de façon à obtenir, dans chaque configuration, pour une pluralité de pixels, un spectre corrigé, les spectres corrigés de chaque configuration étant utilisés pour réaliser une reconstruction tomographique de l'objet. Cela permet d'appliquer l'invention à une reconstruction tomographique

Quel que soit le mode de réalisation, les étapes a) et e) peuvent être complétées par les étapes suivantes :

   f) sélection d'au moins une plage d'énergie ;
   g) réalisation d'une image, dont chaque pixel comprend une donnée obtenue à partir d'un spectre corrigé associé à un pixel du détecteur, dans ladite plage d'énergie.

Lors de cette étape g), chaque pixel de l'image peut comporter une information relative à une intégrale ou à une moyenne dudit spectre corrigé dans ladite plage d'énergie. Il peut également s'agir d'une médiane ou d'un autre indicateur statistique du spectre corrigé dans ladite plage d'énergie, exprimant, relatif à une quantité de photons détectés dans ladite plage d'énergie.

Quel que soit le mode de réalisation, le procédé peut comprendre l'une des caractéristiques suivantes, prises isolément ou en combinaison :

- lors de l'étape d), l'estimation dudit spectre primaire comprend le produit matriciel de ladite matrice de passage par chaque spectre de comparaison ;
- lors de l'étape c), le spectre de comparaison est obtenu, pour chaque pixel, en réalisant une soustraction du premier spectre et du deuxième spectre ;
- chaque élément absorbant est apte à absorber entre 5% et 80% du rayonnement auquel il est exposé, et de préférence moins de 60%, voire 40%, voire 30% de ce rayonnement ;
- le masque définit un facteur de remplissage, correspondant à un ratio entre la surface occupée par l'ensemble des éléments absorbants constituant le masque sur la surface selon laquelle s'étend le masque, ainsi qu'une atténuation globale, correspondant à un produit dudit facteur de remplissage par un pourcentage de rayonnement incident absorbé par le masque, l'atténuation globale du masque étant de préférence comprise entre 1 et 20%, et de préférence entre 1 et 10% ;
- chaque élément absorbant du masque est distant d'un autre élément absorbant d'une distance inférieure à 1 cm, et est de préférence compris entre 1 mm et 5 mm ;
- l'espacement entre deux éléments absorbants adjacents, projetés sur le détecteur, est compris entre 1 et 10 cm, et de préférence inférieur à 5 cm ou 3 cm ;
- le masque est interposé entre la source et l'objet ;
- la matrice de passage est obtenue en réalisant une pluralité de mesures dites de calibration, chaque mesure de calibration étant effectuée en interposant un matériau de nature et d'épaisseur connue, dit matériau de calibration, entre la source d'irradiation et le détecteur. De préférence, la matrice de passage est obtenue en utilisant différents matériaux de calibration, de nature ou d'épaisseur différentes ;
- le détecteur est un détecteur matriciel bidimensionnel ;
- le détecteur est un détecteur semi-conducteur.

Un autre objet de l'invention est un support d'enregistrement d'informations, comportant des instructions pour l'exécution d'un procédé tel que décrit dans cette demande, ces instructions étant aptes à être exécutées par un microprocesseur.

Un autre objet de l'invention est un dispositif pour la réalisation d'images d'un objet comportant :

- une source d'irradiation, apte à émettre un rayonnement électromagnétique ionisant, dit rayonnement incident, vers ledit objet ;
- un détecteur comportant des pixels, chaque pixel étant apte à détecter un rayonnement transmis par l'objet vers le détecteur, et à en acquérir un spectre ;
- un masque, apte à être interposé entre la source et l'objet, ledit masque comportant des éléments absorbants, aptes à absorber une partie dudit rayonnement incident, et dont une projection sur le détecteur définit un premier groupe de pixels ;
- un processeur, apte recevoir des spectres détectés par chaque pixel, et à mettre en oeuvre le procédé décrit dans cette demande.

L'invention sera mieux comprise par les exemples de réalisation décrits ci-après, lesquels se basent sur les figures suivantes.

## FIGURES

Les figures 1A et 1B représentent un dispositif permettant la mise en oeuvre de l'invention. La figure 1C représente un masque équipant le dispositif, également dessiné sur la figure 1A.

La figure 2A représente une matrice de réponse du détecteur. Les figures 2B et 2C représentent respectivement une ligne et une colonne de cette matrice de réponse.

La figure 3 représente les principales étapes d'un mode de réalisation de l'invention.

La figure 4A représente une matrice de passage mise en oeuvre dans l'invention. La figure 4B représente une colonne de cette matrice de passage. La figure 4C représente les étapes d'un procédé permettant l'obtention de cette matrice de passage.

Les figures 5A et 5B représentent un mode de réalisation de l'invention adapté à une reconstruction tomographique d'un objet.

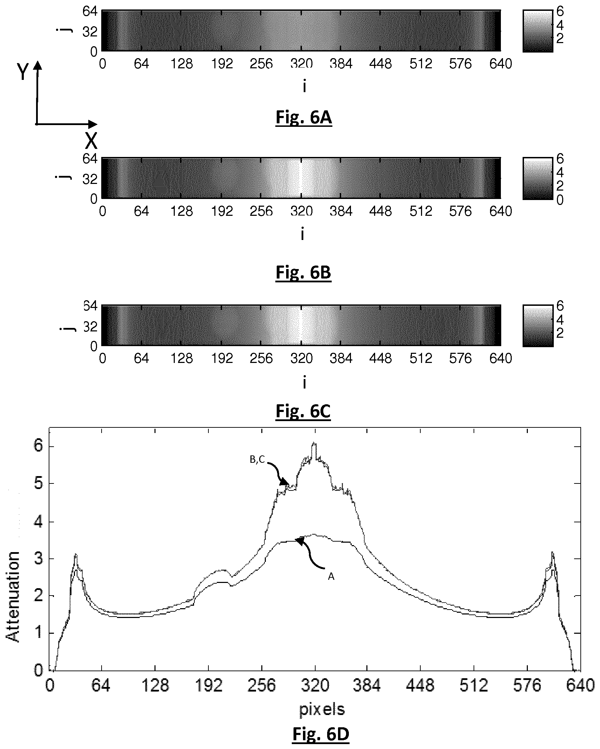

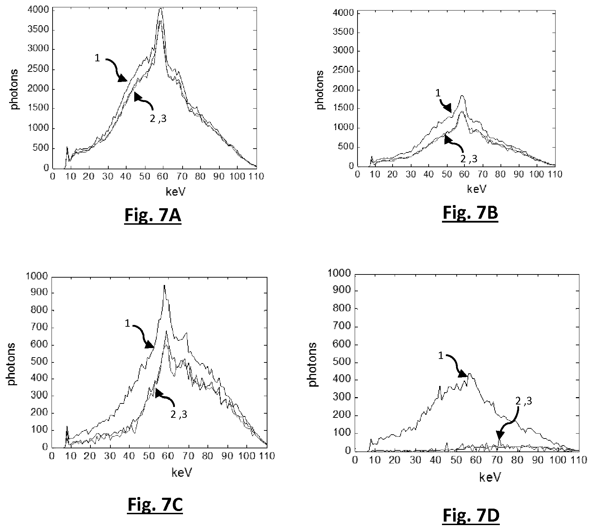

Les figures 6A, 6B, et 6C représentent des résultats de modélisations simulant une image d'un objet, chaque image étant basée sur des spectres mesurés par les pixels d'un détecteur, ces spectres représentant respectivement le rayonnement total, le rayonnement primaire et l'estimation du rayonnement primaire selon l'invention. La figure 6D représente un profil horizontal de chacune des figures 6A, 6B et 6C. Les figures 7A, 7B, 7C et 7D représentent des spectres du rayonnement total, du rayonnement primaire ainsi que du rayonnement primaire estimé selon l'invention, mesurés par différents pixels du détecteur.

Les figures 8A, 8B et 8C représentent des modélisations d'une reconstruction tomographique, dans différentes plages d'énergies, la reconstruction tomographique étant respectivement réalisée sur la base du rayonnement total, du rayonnement primaire et de l'estimation du rayonnement primaire selon l'invention.

Les figures 9A, 9B, et 9C représentent des images de mesures expérimentales réalisées sur un fantôme, chaque image étant basée sur des spectres mesurés par les pixels d'un détecteur, ces spectres représentant respectivement le rayonnement total, le rayonnement primaire estimé selon une méthode de référence et l'estimation du rayonnement primaire selon l'invention. La figure 9D montre des profils de chacune des figures 9A, 9B et 9C selon une ligne représentée sur la figure 9B.

Les figures 10A, 10B, 10C et 10D montrent des spectres en énergie du rayonnement total, du rayonnement primaire estimé selon une méthode de référence et du rayonnement primaire tel qu'obtenu selon l'invention, ces spectres correspondant aux quatre points représentés sur la figure 9B.

## EXPOSE DE MODES DE REALISATION PARTICULIERS

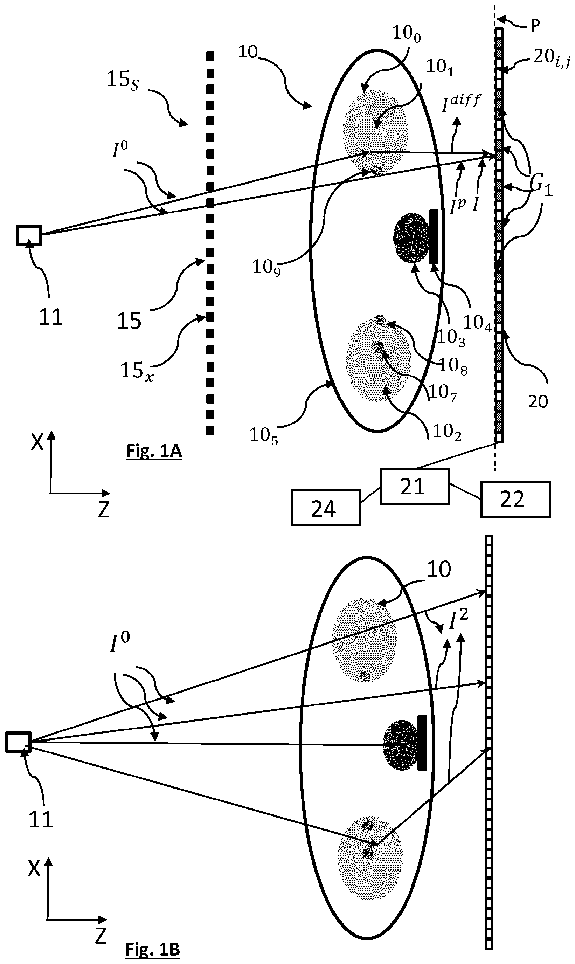

La figure 1A représente un mode de réalisation d'un dispositif 1 mettant en oeuvre un procédé selon l'invention. Une source d'irradiation 11 émet un rayonnement électromagnétique ionisant $I^0$, dit rayonnement incident, vers un objet 10. L'objet 10 est disposé entre la source d'irradiation 11 et un détecteur de rayonnement 20. Le détecteur de rayonnement est un détecteur comprenant des pixels $20_{i,j}$ agencés selon un plan, dit plan de détection P. Les indices $i,j$ désignent les coordonnées de chaque pixel dans le plan de détection. Les pixels peuvent s'étendre selon une ligne mais en général, ils s'étendent selon une matrice régulière bidimensionnelle.

L'objet 10 peut être un tissu biologique vivant, par exemple une partie du corps d'un animal ou d'un être humain. Le dispositif est alors un dispositif d'imagerie médicale. L'objet peut également être une pièce industrielle ou un bagage, le dispositif étant alors utilisé à des fins de contrôle non destructif. Dans cet exemple, l'objet est une partie d'un fantôme représentant un corps humain, comportant une matrice $10_0$, deux poumons $10_1$, $10_2$, une colonne vertébrale $10_3$, une vertèbre $10_4$, des cotes $10_5$ ainsi que des tumeurs, $10_7$, $10_8$ et $10_9$.

Le terme rayonnement électromagnétique ionisant désigne un rayonnement électromagnétique constitué de photons d'énergie supérieure à 1 keV, et de préférence inférieure à 5 MeV. La plage d'énergie du rayonnement ionisant peut être comprise entre 1 keV et 2 MeV, mais elle s'étend le plus souvent entre 1 keV et 150 keV ou 300 keV. Le rayonnement ionisant peut être un rayonnement X ou γ. De préférence, la source de rayonnement ionisant est poly-énergétique, le rayonnement incident étant émis selon une plage d'énergie s'étendant généralement selon plusieurs dizaines voire centaines de keV. Il s'agit notamment d'un tube émetteur de rayons X.

Une partie des photons, constituant le rayonnement incident $I^0$, traversent l'objet et atteignent le détecteur 20, sans interagir avec l'objet. Ces photons forment une composante primaire, ou rayonnement primaire $I^p$. D'autres photons constituant le rayonnement incident $I^0$ sont absorbés dans l'objet, par exemple par effet photoélectrique. Enfin, certains photons subissent une interaction de diffusion dans l'échantillon, de type diffusion inélastique Compton ou diffusion élastique Rayleigh. La diffusion, qu'elle soit inélastique ou élastique, engendre un changement de la direction du photon.

Ainsi, l'objet 10 irradié par la source 11 transmet au détecteur 20 un rayonnement $I,$ dit rayonnement transmis, comportant :

- une composante directe, ou rayonnement primaire, $I^p$, n'ayant pas interagi avec l'objet, et dont la trajectoire depuis la source est rectiligne ;
- une composante de diffusion $I^{diff},$ ou rayonnement de diffusion, due à une diffusion du rayonnement incident dans l'objet.

Le rayonnement transmis $I$ par l'objet atteint les pixels du détecteur 20, chaque pixel détectant une partie de ce rayonnement. Le rayonnement transmis par l'objet et détecté par un pixel $20_{i,j}$ est noté $I_{i,j}$.

Comme évoqué en relation avec l'art antérieur, le rayonnement de diffusion $I^{diff}$ perturbe l'interprétation des mesures. En effet, contrairement au rayonnement primaire $I^p$, le rayonnement de diffusion se propage depuis l'objet vers le détecteur, selon une direction variable. Ainsi, une partie du rayonnement collecté par chaque pixel $20_{i,j}$ du détecteur ne provient non pas directement de la source de rayonnement 11, mais résulte du phénomène de diffusion. Or, l'interprétation des images est basée sur l'atténuation du rayonnement incident par le détecteur, cette dernière étant obtenue par un ratio, sur une plage d'énergie donnée, entre l'intensité du rayonnement primaire $I^p$ sur l'intensité du rayonnement incident $I^0$. Une bonne interprétation des images suppose la connaissance de l'intensité du rayonnement primaire $I^p$, alors que le rayonnement $I$ transmis par l'objet, et mesuré par le détecteur, comporte une somme dudit rayonnement primaire $I^p$ et du rayonnement diffusé $I^{diff}$.

Chaque pixel $20_{i,j}$ constitue un détecteur de rayonnement, comportant :

- un matériau détecteur, apte à interagir avec les photons du rayonnement $I$ transmis par l'objet, ce matériau étant de type scintillateur ou, de préférence, un matériau semi-conducteur compatible avec une utilisation à la température ambiante, de type CdTe, CdZnTe ;
- un circuit électronique, apte à générer un signal dont l'amplitude dépend, et est de préférence proportionnelle, à l'énergie déposée par chaque photon interagissant dans le matériau détecteur ;
- un circuit de spectrométrie, apte à établir un spectre, noté $\boldsymbol{S_{i,j}}$ en énergie des signaux détectés pendant une période temporelle, dite période d'acquisition.

Ainsi, lorsque les pixels sont disposés régulièrement selon un agencement matriciel, chaque pixel est apte à produire un spectre $\boldsymbol{S_{i,j}}$ du rayonnement transmis par l'objet. Le détecteur est alors apte à former plusieurs images, chaque image représentant un contenu de chaque spectre dans une plage d'énergie $\Delta E$ déterminée. Typiquement, chaque image comporte l'intégrale ou la valeur moyenne de chaque spectre $\boldsymbol{S_{i,j}}$ dans ladite bande d'énergie. On parle alors d'imagerie spectrale, puisque le détecteur est à la fois résolu spatialement et spectralement.

Aussi, sous l'effet de l'irradiation par le rayonnement incident $I^0$, l'objet 10 transmet un rayonnement $I$, dit rayonnement transmis, vers un détecteur spectrométrique pixellisé 20, dont chaque pixel $20_{i,j}$ est apte détecter ledit rayonnement transmis $I$ et à former un spectre en énergie $\boldsymbol{S_{i,j}}$ du rayonnement $I_{i,j}$ ainsi détecté.

Le terme spectre en énergie correspond à un histogramme de l'amplitude des signaux détectés au cours d'une période d'acquisition du spectre. Une relation entre l'amplitude A et l'énergie E peut être obtenue par une fonction d'étalonnage en énergie $g$ telle que $E = g(A),$ selon des principes connus de l'homme du métier. Un spectre d'énergie $\boldsymbol{S_{i,j}}$ est donc un vecteur, dont chaque terme $S_{i,j}(n)$ représente une quantité de rayonnement détecté par le pixel $20_{i,j}$

dans une plage d'énergie $E \pm \frac{\partial E}{2},$ avec $\partial E$ étant la largeur spectrale de chaque canal.

Chaque spectre en énergie $\boldsymbol{S_{i,j}}$ peut être considéré comme une somme d'un spectre du rayonnement primaire, noté $S_{i,j}^p$ et d'un spectre du rayonnement de diffusion $S_{i,j}^{diff}$, à un terme de bruit près. Aussi, $S_{i,j} \approx S_{i,j}^p + S_{i,j}^{diff}$ (1). Le signe $\approx$ signifie une égalité à un terme de bruit près, ce bruit pouvant notamment résulter du détecteur ou d'effets dits d'empilement, se produisant lorsque deux photons incidents sont détectés simultanément.

Un objectif de l'invention est de corriger le spectre mesuré par chaque pixel, de façon à réduire la composante de diffusion $S_{i,j}^{diff}$ et d'établir un spectre corrigé $S_{i,j}^*$ tel que $S_{i,j}^* \approx S_{i,j}^p$. Autrement dit, le spectre corrigé $S_{i,j}^*$ correspond à une estimation $\widehat{S}_{i,j}^p$ du spectre du rayonnement primaire atteignant le pixel $20_{i,j}$ : $S_{i,j}^* = \widehat{S}_{i,j}^p$.

Le dispositif comporte également un masque 15, disposé entre la source 11 et le détecteur 20 et dans cet exemple, entre la source 11 et l'objet 10, ce qui constitue la configuration préférée. Ce masque comporte des éléments absorbants $15_x$ répartis spatialement selon une surface $15_s$ selon laquelle s'étend le masque. Chaque élément absorbant est apte à absorber partiellement une partie du rayonnement incident $I^0$ produit par la source d'irradiation. Les éléments absorbants sont répartis discrètement, de telle sorte que l'espace entre deux éléments absorbants adjacents est moins absorbant que lesdits éléments absorbants. Autrement dit, les éléments absorbants définissent une répartition spatiale discrète de coefficients d'atténuation $att_{15}^x$, $att_{15}^{x\prime}$ de telle sorte qu'entre deux éléments absorbants adjacents $15_x$, $15_{x\prime}$, le coefficient d'atténuation $att_{15}^0$ est inférieur au coefficient d'atténuation $att_{15}^x$, $att_{15}^{x\prime}$ associé à chaque élément absorbant.

Le terme coefficient d'atténuation est connu de l'homme du métier. Il peut être exprimé selon l'expression $att_{15}^x(E) = -ln\left[\frac{I^x(E)}{I^0(E)}\right]$, où $I^0(E)$ désigne une intensité, à une énergie $E$, d'un rayonnement incident $I^0$ à un l'élément absorbant $15_x$ et $I^x(E)$ désigne une intensité, à ladite énergie $E$ d'un rayonnement $I^x$ transmis par l'élément absorbant $15_x$.

D'une façon générale, l'interposition du masque entre la source et le détecteur ne doit pas modifier significativement le rayonnement de diffusion parvenant au détecteur, par rapport à une configuration sans masque. Aussi, de préférence, chaque élément absorbant présente un coefficient d'atténuation, tel que précédemment défini, compris entre 0.05 et 1.5, à une des énergies de la plage d'énergie selon laquelle est émis le rayonnement incident $I^0$, ou à l'énergie moyenne de cette plage d'énergie. Ainsi, en négligeant la diffusion, chaque élément absorbant absorbe, de préférence, entre 5 % et 80% du rayonnement incident $I^0$ produit par la source et/ou traversant le masque dans l'espace s'étendant entre les éléments absorbants du masque. De préférence, le coefficient d'atténuation est inférieur à 1 voire inférieur à 0.5 et de préférence inférieur à 0.3. Ainsi, chaque élément absorbant absorbe respectivement moins de 60 %, ou moins de 40%, et de préférence moins de 30% du rayonnement produit par la source, ou du rayonnement passant entre les éléments absorbants du masque. En dessous d'un coefficient d'atténuation égal à 0.05, correspondant à une atténuation de 5% du rayonnement produit par la source, les inventeurs considèrent que l'atténuation n'est pas suffisante. Autrement dit, le masque 15 permet donc établir un contraste d'atténuation, entre les éléments absorbants $15_x$ et l'espace s'étendant entre lesdits éléments absorbant, ces derniers absorbant entre 5% et 30%, voire 40%, voire davantage du rayonnement traversant ledit espace.

En complément ou alternativement, on peut définir une atténuation globale du masque 15 sous la forme d'un produit d'un facteur de remplissage par le pourcentage du rayonnement incident absorbé par le masque, ce dernier étant déterminé à une énergie de la plage d'énergie du rayonnement incident $I^0$ émis par la source d'irradiation 11, ou à une énergie moyenne de cette plage. Le facteur de remplissage correspond à un ratio entre la surface du masque occupée par l'ensemble des éléments absorbants $15_x$ et la surface totale du masque. L'atténuation globale du masque, ainsi définie, est de préférence supérieure à 1% et inférieure à 10%. Ainsi, un masque satisfaisant à cette condition peut avoir un facteur de remplissage égal à 0.08, chaque élément $15_x$ du masque absorbant 10% du rayonnement incident, ce qui donne une atténuation globale du masque, telle que précédemment définie, égale à 0.08 (8%).

Chaque élément absorbant peut avoir une forme quelconque mais au moins une dimension, dans une direction de la surface $15_s$ selon lequel il s'étend, est inférieure à 5 mm, et de préférence inférieure à 2 mm voire à 1 mm. Dans l'ensemble des modes de réalisation précédemment décrits, le masque s'étend de préférence selon un plan XY parallèle à un plan selon lequel s'étendent les pixels du détecteur.

L'espacement entre deux éléments absorbants adjacents, au niveau du masque, peut être inférieur à 5 mm, et est de préférence compris entre 1 mm et 5 mm. D'une façon générale, l'espacement entre deux éléments absorbants adjacents, après projection sur le détecteur 20, est avantageusement compris entre 1 et 10 cm, et de préférence inférieur à 5 cm ou à 3 cm. Comme décrit par la suite, la projection de chaque élément absorbant $15_x$ sur le détecteur définit une zone d'ombre élémentaire. Chaque zone d'ombre élémentaire s'étend autour d'un point central. Avantageusement, l'espacement entre les points centraux de deux zones d'ombre élémentaires adjacentes est compris entre 1 et 10 cm, et de préférence compris entre 1cm et 5 cm. Par projection, on entend une projection selon la direction de propagation du rayonnement émis par la source.

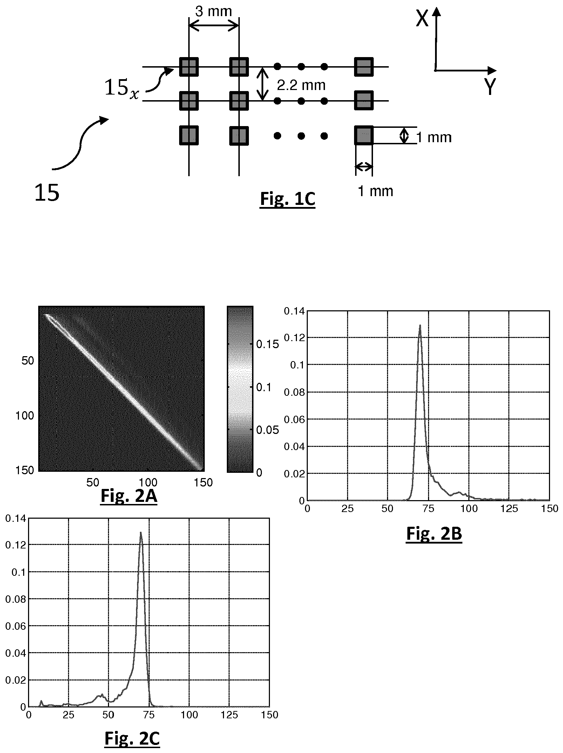

Un exemple de masque est représenté sur la figure 1C. Chaque élément absorbant $15_x$ est un plot parallélé-pipédique, dont l'aire, selon la surface $15_s$ selon laquelle s'étend le masque, est de 1 mm * 1 mm, l'espacement entre le centre de chaque élément $15_x$ étant respectivement de 3 mm selon une première direction Y et 2.2 mm selon une deuxième direction X perpendiculaire à la première direction. Dans cet exemple, le matériau constituant le masque est de l'Aluminium, et son épaisseur est de 2 mm, ce qui correspond à un coefficient d'atténuation de 0.9 à 100 keV, ce qui conduit à atténuer 10% du rayonnement incident. L'épaisseur s'entend selon une direction perpendiculaire à la surface selon laquelle s'étend le masque. D'une façon générale, le masque est réalisé selon un matériau suffisamment absorbant pour atténuer une portion suffisante du rayonnement primaire. On évite toutefois les matériaux trop denses, de type métaux lourds, par exemple le plomb, susceptibles de produire une diffusion significative du rayonnement incident avant que ce dernier n'atteigne l'objet. Parmi les matériaux préférés composant le masque, on peut citer l'aluminium, le cuivre, le graphite, le bore.

D'autres géométries sont envisageables, en considérant par exemple un espacement non régulier entre les différents éléments absorbants, ou une géométrie non régulière de chaque élément absorbant. Un masque en forme de grille, définissant des mailles, est également possible, les éléments absorbants étant disposés entre chaque maille.

La surface $15_s$ selon laquelle s'étend le masque, entre chaque élément absorbant, est de préférence constituée d'un matériau considéré comme transparent à l'égard des photons, dans la plage d'énergie considérée. Il peut s'agir d'une fine épaisseur d'un plastique, d'un papier ou d'un métal léger, de type aluminium, fer, cuivre, ou un espace laissé libre et occupé par de l'air. Ainsi, entre chaque élément absorbant $15_x$, le coefficient d'atténuation, tel que précédemment défini, est de préférence inférieur à 0.5, voire à 0.2 ou encore 0.1. De préférence, entre chaque élément absorbant, le coefficient d'atténuation est négligeable.

Le nombre d'éléments absorbants est dimensionné de façon à couvrir le champ d'observation du détecteur. Dans l'exemple décrit, les éléments absorbants sont répartis selon une matrice de 17 par 3 éléments, soit un total de 51 éléments.

Le masque peut être amovible ou fixe. Le fait de disposer d'un masque amovible permet la réalisation d'acquisitions de spectre sans le masque, comme cela est représenté sur la figure 1B.

Lorsque le masque est interposé entre la source et le détecteur, sa projection, sur le détecteur, selon la direction de propagation du rayonnement incident $I^0$, définit une zone d'ombre, rassemblant les pixels du détecteur $20_{i,j}^x$ alignés par rapport à chaque élément absorbant $15_x$, selon ladite direction de propagation. Plus précisément, comme précédemment évoqué, la projection de chaque élément absorbant $15_x$, selon la direction de propagation du rayonnement incident, forme une zone d'ombre élémentaire sur le détecteur. L'ensemble des zones d'ombre élémentaires constitue ladite zone d'ombre. Les pixels de la zone d'ombre constituent un premier groupe de pixel, noté $G_1$. Ce premier groupe $G_1$ peut être déterminé préalablement soit par calcul, soit expérimentalement, sans objet entre la source et le détecteur.

Les pixels $20_{i,j \notin G1}^x$ n'appartenant pas à ce premier groupe reçoivent un rayonnement non atténué par les éléments absorbants $15_x$, tandis que chaque pixel $20_{i,j \in G1}^x$ appartenant à ce premier groupe reçoit un rayonnement atténué par un élément absorbant $15_x$, ce dernier étant situé sur une ligne s'étendant entre ledit pixel et la source d'irradiation 11.

Le dispositif comprend également une unité de calcul, ou processeur 21, par exemple un microprocesseur, est apte à traiter chaque spectre $S_{i,j}$ mesuré par les pixels du détecteur. En particulier, le processeur est un microprocesseur relié à une mémoire programmable 22 dans laquelle est stockée une séquence d'instructions pour effectuer les opérations de traitement de spectres et de calculs décrites dans cette description. Ces instructions peuvent être sauvegardées sur un support d'enregistrement, lisible par le processeur, de type disque dur, CDROM ou autre type de mémoire. Le processeur peut être relié à une unité d'affichage 24, par exemple un écran.

Le détecteur peut être caractérisé par une matrice de réponse **D** du détecteur, représentant les imperfections de la détection. Cette matrice, de taille $N \times N$, $N$ désignant le nombre de canaux de chaque spectre formé par le détecteur, est représentée sur la figure 2A. Dans cet exemple, $N = 150$, mais de façon générale, le nombre de canaux est supérieur à 2, voire supérieur à 10, et peut atteindre plusieurs milliers. Chaque terme $D(u, v)$ de cette matrice représente une probabilité qu'un photon incident au détecteur, d'énergie $v$, soit considéré par le détecteur comme ayant une énergie $u$.

Autrement dit, chaque ligne $D(u, \cdot)$ de la matrice, telle que celle représentée sur la figure 2B, correspond à une distribution de probabilité de l'énergie $v$ d'un photon atteignant le détecteur lorsqu'un photon d'énergie $u$ est détecté. La figure 2B représente la ligne 70 de la matrice $D$. De façon analogue, chaque colonne $D(\cdot, v)$ de la matrice, telle que celle représentée sur la figure 2C, représente une distribution de probabilité de l'énergie détectée $u$ par le détecteur lorsque le photon atteignant le détecteur a une énergie $v$. La figure 2C représente la colonne 70 de la matrice $D$. Plus résolution en énergie du détecteur est fine, plus cette matrice tend vers une matrice diagonale. Dans le cas d'un détecteur parfait, la matrice $D$ est la matrice identité.

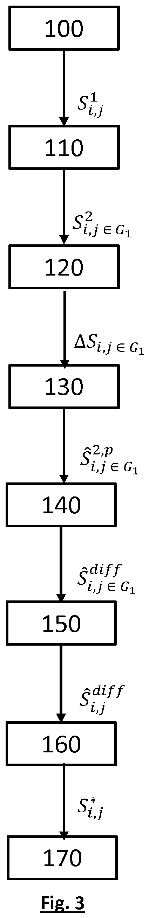

On va à présent décrire les principales étapes d'un procédé selon un premier mode de réalisation de l'invention, en lien avec la figure 3.

Etape 100 : acquisition d'un premier spectre. Le masque 15 est interposé entre la source 11 et le détecteur 20. Chaque pixel $20_{i,j}$ est exposé à un rayonnement $I^1$, dit premier rayonnement, transmis par l'objet en présence du masque, et en acquiert un spectre, dit premier spectre, $S^1_{i,j}$. Les pixels $20_{i,j \in G_1}$, situés dans le prolongement des éléments absorbants $15_x$, appartiennent au premier groupe $G_1$ et détectent un rayonnement atténué par rapport aux autres pixels $20_{i,j \notin G_1}$. Le premier spectre acquis par les pixels du premier groupe est noté $S^1_{i,j \in G_1}$. Le premier spectre acquis par les pixels n'appartenant pas au premier groupe est noté $S^1_{i,j \notin G_1}$

Etape 110 : obtention, pour chaque pixel $20_{i,j \in G_1}$ du premier groupe $G_1$, d'un deuxième spectre $S^2_{i,j \in G_1}$ représentatif d'un deuxième rayonnement $I^2$ transmis par l'objet sans masque interposé entre la source et l'objet. Ce deuxième spectre peut être obtenu expérimentalement, selon la configuration représentée sur la figure 1B, le masque 15 étant retiré. Ce deuxième spectre peut également être obtenu par une estimation, à partir des premiers spectres $S^1_{i,j \notin G_1}$ n'appartenant pas au premier groupe $G_1$, et recevant un rayonnement non atténué par les éléments absorbants. Cette estimation peut être réalisée par interpolation, par exemple une interpolation linéaire ou polynomiale. Elle permet d'estimer le deuxième spectre $S^2_{i,j \in G_1}$ reçu par les pixels du premier groupe, en l'absence de masque, sans avoir à réaliser une mesure expérimentale.

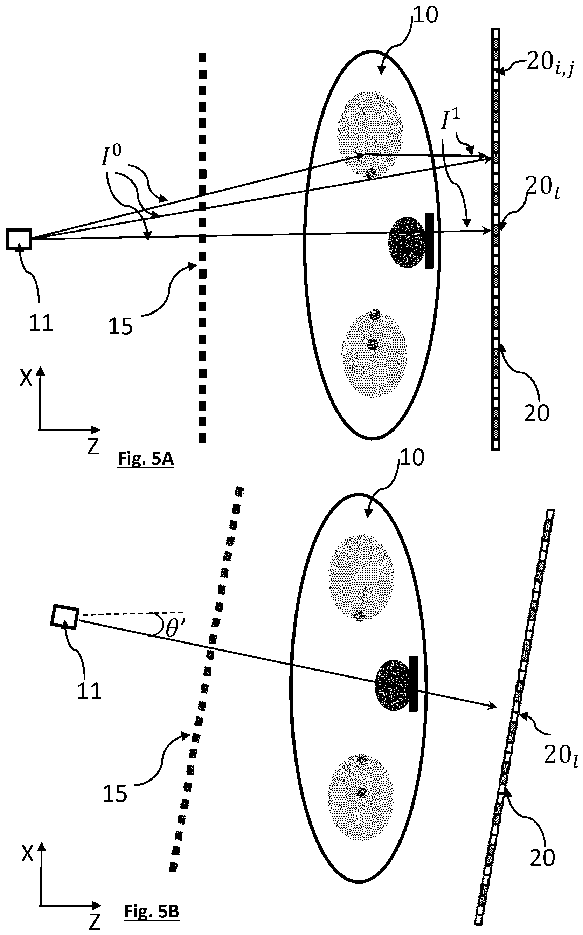

Selon un autre mode de réalisation, détaillé ultérieurement, en lien avec les figures 5A et 5B, à chaque mesure correspond une configuration de mesure $C$, représentative de la position du détecteur et/ou de la source vis-à-vis de l'objet. Cette configuration de mesure C évolue entre deux mesures successives. Le deuxième spectre $S^{2,C}_{i,j \in G_{1,C}}$ reçu par un pixel du premier groupe le détecteur, selon une configuration $C$, peut être estimé à partir d'un premier spectre $S^{1,C'}_{i,j \notin G_{1,C'}}$, réalisé lors d'une autre configuration $C'$, dans laquelle ledit pixel n'est pas dans le prolongement d'un élément absorbant et n'appartient pas, dans cette autre configuration, au premier groupe $G_{1,C'}$. Ce cas de figure est exposé plus en détail ultérieurement, lors de la description des figures 5A et 5B.

Etape 120 : pour chaque pixel $20_{i,j \in G1}$ du premier groupe $G_1$, détermination d'un spectre dit de comparaison $\Delta S_{i,j \in G1}$, obtenu par une comparaison, pour le même pixel, entre le premier spectre $S^1_{i,j \in G_1}$ et le deuxième spectre $S^2_{i,j \in G_1}$. Par exemple, la comparaison est une soustraction, auquel cas $\Delta S_{i,j \in G_1} = S^2_{i,j \in G_1} - S^1_{i,j \in G_1}$ (2). Par soustraction de spectres, il est entendu une soustraction du contenu de chaque canal, de type soustraction vectorielle.

Etape 130 : estimation d'un spectre dit primaire $\widehat{S}^{2,p}_{i,j \in G_1}$ pour chaque pixel du premier groupe, sans masque disposé entre ledit pixel et la source. Un tel spectre représente la composante primaire du deuxième spectre $S^2_{i,j \in G_1}$ atteignant un pixel du premier groupe $G_1$. Cette estimation est réalisée, pour chaque pixel de ce groupe, en considérant le spectre de comparaison $\Delta S_{i,j \in G1}$ déterminé lors de l'étape 120, par le biais du produit matriciel :

$$\widehat{S}^{2,p}_{i,j \in G_1} = M \times \Delta S_{i,j \in G_1} \ (3)$$

$M$ est une matrice de passage, préalablement déterminée, établissant un lien entre le spectre de comparaison, déterminé lors de l'étape 120, et une estimation $\widehat{S}^{2,p}_{i,j \in G_1}$ de la composante primaire du rayonnement $I^2$ atteignant le pixel $20_{i,j \in G_1}$ du premier groupe en l'absence de masque.

Cette estimation constitue un aspect important de l'invention. Elle trouve son origine en explicitant les spectres

$S^1_{i,j\,\epsilon\,G_1}$, $S^2_{i,j\,\epsilon\,G_1}$ mesurés par un pixel $20_{i,j\in G_1}$ du premier groupe respectivement avec et sans masque. Elle se base sur une hypothèse selon laquelle l'évolution spectre du rayonnement diffusé $S^{diff}_{i,j\,\epsilon\,G_1}$, avec ou sans masque, est négligeable. Sa validité est donc conditionnée par l'utilisation d'un masque absorbant et peu diffusant tel que précédemment décrit.

En se basant sur l'expression (1), $S^1_{i,j\,\epsilon\,G_1}$ peut être exprimé selon la forme :

$$S^1_{i,j\,\epsilon\,G_1} = S^{1\,p}_{i,j\,\epsilon\,G_1} + S^{1diff}_{i,j\,\epsilon\,G_1} \ (4),$$

$S^{1\,p}_{i,j\,\epsilon\,G_1}$ et $S^{1diff}_{i,j\,\epsilon\,G_1}$ représentant respectivement les spectres directs et de diffusion du spectre $S^1_{i,j\,\epsilon\,G_1}$.

$$S^{1\,p}_{i,j\,\epsilon\,G_1} = D \times \left(S^0 \circ e^{-att_{10}-att_{15}}\right) \ (5)$$

où : $D$ désigne la matrice de réponse du détecteur, précédemment décrite, $S^0$ désigne le spectre du rayonnement incident $I^0$, $att_{10}$ et $att_{15}$ désignent respectivement l'atténuation du rayonnement atteignant le pixel $20_{i,j}$ dans l'objet 10 et dans le masque 15 à chaque énergie du spectre. $att_{10} = \sum_q l_q\,\mu_q$, et $att_{15} = l_{15x}\mu_{15x}$ où $l_q$ désigne la longueur parcourue dans chaque élément $10q$ de l'objet 10, $\mu_q$ désigne un vecteur de coefficients d'atténuation linéaire, à chaque énergie du spectre, de chaque élément $10q$, $l_{15x}$ désigne la longueur parcourue dans l'élément absorbant $15_x$ conjugué au pixel $20_{i,j}$ et $\mu_{15x}$ désigne un vecteur de coefficient d'atténuation linéaire à chaque énergie du spectre. $\times$ désigne le produit matriciel et $\circ$ désigne le produit de Hadamard. Un coefficient d'atténuation linéaire $\mu_q$ répond à la définition suivante :

$l_q\mu_q(E) = -ln\left[\frac{I^q(E)}{I^{0,q}(E)}\right]$, $I^{0,q}$ et $I^q$ désignant respectivement le rayonnement incident et transmis par un élément $10q$ de l'objet d'épaisseur $l_q$. Un tel coefficient d'atténuation linéaire, connu de l'homme du métier, dépend de l'énergie et des matériaux constituant l'élément $10q$.

$$S^2_{i,j\,\epsilon\,G_1} = S^{2\,p}_{i,j\,\epsilon\,G_1} + S^{2diff}_{i,j\,\epsilon\,G_1} \ (6),$$

avec

$$S^{2\,p}_{i,j\,\epsilon\,G_1} = D \times \left(S^0 \circ e^{-att_{10}}\right) \ (7)$$

car le spectre $S^2_{i,j\,\epsilon\,G_1}$ correspond à une acquisition sans écran atténuateur 15.

Il vient donc :

$$\Delta S_{i,j\in G_1} = S^2_{i,j\,\epsilon\,G_1} - S^1_{i,j\,\epsilon\,G_1} = D \times \left(S^0 \circ e^{-att_{10}}\right) - D \times \left(S^0 \circ e^{-att_{10}-att_{15}}\right) \ (8)$$

car par hypothèse, $S^{2diff}_{i,j\,\epsilon\,G_1} = S^{1diff}_{i,j\,\epsilon\,G_1}$.

Donc

$$\Delta S_{i,j\in G_1} = D \times \left[(1 - e^{-att_{15}}) \circ \left(S^0 \circ e^{-att_{10}}\right)\right] \ (9),$$

1 est un vecteur ne comportant que des 1.

d'où

$$\Delta S_{i,j \in G_1} = D \times \left[ (1 - e^{-att_{15}}) \circ S^{2\,p}_{i,j\,\epsilon\,G_1} \right] (10)$$

donc

$$\Delta S_{i,j \in G_1} = D \times w S^{2\,p}_{i,j\,\epsilon\,G_1} \quad (12)$$

avec

$$w = (1 - e^{-att_{15}}) \,(13).$$

On peut constituer une matrice, noté $W^{-1}$ carrée, de dimension $N$ par $N$, les termes de cette matrice n'étant

$$W^{-1}(n,n) = \frac{1}{(1 - e^{-att_{15}(n)})},$$

pas situés sur la diagonale étant nuls, et chaque terme $n$ de la diagonal ayant pour valeur
(14) où $att_{15}$(n) désigne l'atténuation de l'écran 15 à l'énergie correspondant au canal de rang $n$. L'indice $n$ désigne le rang d'un canal du spectre, ce qui peut être assimilé à une valeur d'énergie $E$. L'atténuation correspond à l'épaisseur d'un élément absorbant multipliée par le coefficient d'atténuation linéaire du matériau constituant le matériau absorbant.
Ainsi, selon les expressions (13) et (14), ayant déterminé, pour un pixel $20_{i,j\in G_1}$, le spectre de comparaison $\Delta S_{i,j\in G_1}$, on peut estimer le spectre primaire $\widehat{S}^{2\,p}_{i,j\,\epsilon\,G_1}$ à l'aide de la matrice de réponse du détecteur $D$ et de la matrice $W^{-1}$ préalablement définie, selon l'expression :

$$\widehat{S}^{2\,p}_{i,j\,\epsilon\,G_1} = D \times W^{-1} \times D^{-1} \times \Delta S_{i,j \in G_1} \quad (15)$$

Cette expression peut être réécrite de la façon suivante : $\widehat{S}^{2\,p}_{i,j\,\epsilon\,G_1} = M \times \Delta S_{i,j\in G_1}$ , ce qui correspond à l'expression (3) précédemment écrite.
Où $M$ est une matrice de passage, telle *que*

$$M = D \times W^{-1} \times D^{-1} \quad (17)$$

La matrice de passage $M$, élaborée à partir de la matrice $D$ de réponse du détecteur et d'une matrice prenant en compte l'atténuation du masque 15, permet d'estimer le spectre primaire $\widehat{S}^{2\,p}_{i,j\,\epsilon\,G_1}$ d'un rayonnement atteignant, sans atténuation, un pixel $20_{i,j\in G_1}$ du premier groupe, à partir d'une comparaison ($\Delta S_{i,j\in G_1}$) entre les spectres respectivement déterminés avec $(S^{1}_{i,j\,\epsilon\,G_1})$ et sans atténuation $(S^{2}_{i,j\,\epsilon\,G_1})$. Dans cet exemple, la comparaison est une différence entre ces deux spectres. L'utilisation de cette matrice de passage est un élément important de l'invention car elle permet de remonter analytiquement à une estimation du spectre primaire sans atténuation à partir du spectre de comparaison $\Delta S_{i,j}$.
La matrice de passage $M$ peut être déterminée analytiquement mais les inventeurs ont proposé une méthode permettant de l'estimer expérimentalement, décrite ci-après.
Etape 140 : estimation du spectre diffusé $\widehat{S}^{diff}_{i,j\,\epsilon\,G_1}$ pour chaque pixel $20_{i,j\in G_1}$ du premier groupe.

Le spectre $\widehat{S}^{2\,p}_{i,j\,\epsilon\,G_1}$ étant estimé pour chaque pixel $20_{i,j\in G_1}$ du premier groupe, à partir de $M$ et de $\Delta S_{i,j\in G_1}$, il est possible d'estimer le spectre de diffusion affectant chaque pixel $20_{i,j\in G_1}$ de ce groupe, en utilisant (6) :

$$\widehat{S}^{2\,diff}_{i,j\,\epsilon\,G_1} = \widehat{S}^{1\,diff}_{i,j\,\epsilon\,G_1} = \widehat{S}^{diff}_{i,j\,\epsilon\,G_1} = S^{2}_{i,j\,\epsilon\,G_1} - \widehat{S}^{2\,p}_{i,j\,\epsilon\,G_1} \,(18).$$

Etape 150 : estimation du spectre diffusé $\widehat{S}^{diff}_{i,j}$ pour chaque pixel $20_{i,j}$ du détecteur. Ayant estimé le spectre de diffusion de chaque pixel $20_{i,j \in G_1}$ du premier groupe, on se base sur une hypothèse selon laquelle l'évolution spatiale du rayonnement diffusé est de basse fréquence. Autrement dit, les fluctuations du spectre du rayonnement diffusé d'un pixel à un autre pixel voisin n'évolue pas de façon brutale. On peut alors estimer le spectre de diffusion $\widehat{S}_{i,j}^{diff}$ de l'ensemble des pixels du détecteur par interpolation des spectres diffusés $\widehat{S}_{i,j \in G_1}^{diff}$ des pixels du premier groupe $G_1$. Pour les pixels n'étant pas dans le premier groupe, le spectre de diffusion est naturellement le même avec et sans masque, puisque ces pixels ne subissent pas l'atténuation du masque.

Préalablement à l'interpolation, on peut effectuer certains prétraitements optionnels. Soient $20_{i,j \in k}$ les pixels disposés à l'ombre d'un même élément absorbant $15_k$. Ces pixels forment un noyau $k$ dont un pixel $20_k$, dit pixel central du noyau $k,$ constitue le centre. On peut estimer un spectre de diffusion $\overline{S}_k^{diff}$ représentatif de ce noyau et l'attribuer au pixel central. Ce spectre représentatif du noyau $k$ est tel que $\overline{S}_k^{diff} = \frac{1}{C_k} \sum 20_{i,j \in k} \widehat{S}_{i,j}^{diff}$ (19), où $C_k$ désigne le nombre de pixels du noyau $k$. Le spectre $\overline{S}_k^{diff}$ représente une moyenne des estimations des spectres de diffusion des pixels du noyau $k$. L'interpolation peut être réalisée en considérant chaque spectre $\overline{S}_k^{diff}$ représentatif de chaque noyau $k$.

Un autre prétraitement optionnel consiste à une mise à l'échelle de chaque spectre $\overline{S}_k^{diff}$ représentatif de chaque noyau $k,$ en se basant sur une hypothèse selon laquelle le spectre de diffusion atteignant l'ensemble des pixels du détecteur a la même forme, c'est-à-dire est similaire d'un pixel à un autre. Cette hypothèse se justifie d'autant plus que la surface du détecteur est petite, typiquement quelques cm$^2$ ou moins. Cette mise à l'échelle consiste à appliquer un scalaire $a_{k,n}$ à chaque canal $n$ d'un spectre de diffusion $\overline{S}_k^{diff}$ représentatif d'un noyau $k$, ce scalaire étant déterminé selon l'expression :

$$a_{k,n} = \underset{a}{\arg\min} \left[ a \left( \frac{1}{K} \sum_{k=1}^{k=K} \bar{S}_k^{diff}(n) \right) - \bar{S}_k^{diff}(n) \right]^2 (20)$$

$K$ représente le nombre de de noyaux $k.$ On peut alors ajuster le spectre $\overline{S}_k^{diff}$ représentatif de la diffusion dans chaque noyau $k$ selon de manière à obtenir un spectre ajusté, noté $\overline{\overline{S}}_k^{diff}$ avec, pour chaque canal $n$,

$$\overline{\overline{S}}_k^{diff}(n) = \frac{a_{k,n}}{K} \sum_{k=1}^{k=K} \bar{S}_k^{diff}(n) \quad (21).$$

Les pixels du détecteur étant agencés selon deux dimensions XY, l'interpolation se fait, selon les variantes utilisées, sur la base des spectres $\widehat{S}_{i,j}^{diff}$, $\overline{S}_k^{diff}$ ou $\overline{\overline{S}}_k^{diff}$ dans une direction X, puis dans l'autre direction Y, ou, simultanément, dans les deux directions X et Y. L'interpolation est réalisée canal d'énergie par canal d'énergie. Elle peut être polynomiale. Dans l'exemple considéré, le détecteur comprend 64 * 640 pixels. L'interpolation est réalisée selon un polynôme d'ordre 4 selon chaque ligne de 640 pixels, puis selon un polynôme d'ordre 2 selon chaque colonne de 64 pixels.

Etape 160 : correction du deuxième spectre $S_{i,j}^2$ (ou du premier spectre $S_{i,j}^1$, pour les pixels n'appartenant pas au premier groupe) pour tout ou partie des pixels $20_{i,j}$ du détecteur, de façon à obtenir un spectre corrigé $S_{i,j}^*$, représentatif du spectre du rayonnement primaire transmis par l'objet. Autrement dit, si $S_{i,j}^p$ désigne le spectre d'un rayonnement primaire transmis par l'objet sur le pixel $20_{i,j}$, $S_{i,j}^* = \widehat{S}_{i,j}^p$.

Cette étape se fait simplement, par une soustraction, pour chaque pixel $20_{i,j}$, de l'estimation du spectre diffusé $\widehat{S}_{i,j}^{diff}$ au deuxième spectre (ou au premier spectre pour les pixels n'appartenant pas au premier groupe).

Ainsi, pour les pixels $20_{i,j \in G_1}$ du premier groupe :

$$S_{i,j}^{*} = \widehat{S}_{i,j}^{p} = S_{i,j}^{2} - \widehat{S}_{i,j}^{diff} (22) ,$$

et pour les pixels $20_{i,j \notin G_1}$ n'appartenant pas au premier groupe :

$$S_{i,j}^{*} = \widehat{S}_{i,j}^{p} = S_{i,j}^{2} - \widehat{S}_{i,j}^{diff} = S_{i,j}^{1} - \widehat{S}_{i,j}^{diff} (22').$$

On obtient alors, pour chaque pixel du détecteur, un spectre $S_{i,j}^{*}$ représentant une estimation du spectre du rayonnement primaire transmis par l'objet. On peut alors obtenir une image $Im(E)$ représentant l'intensité du rayonnement primaire détecté par chaque pixel, et cela à une ou une pluralité de d'énergies ($E$), d'où la désignation d'image spectrale. C'est sur la base de cette image spectrale que la mesure est interprétée.

Dans le cas d'une reconstruction tomographique, le procédé est mis en oeuvre dans différentes configurations $C$, telles que précédemment décrites, à chaque configuration correspondant une ou une pluralité d'images spectrales $Im^c(E)$, sur la base desquelles la reconstruction tomographique est réalisée.

Quel que soit le mode de réalisation, différents canaux $E$ peuvent être regroupés pour constituer des bandes spectrales $\Delta E$. On peut alors obtenir une image $Im(\Delta E)$ pour chacune de ces bandes spectrales.

Etablissement de la matrice de passage.

Un des éléments clefs de l'invention est la matrice de passage $M$ utilisée lors de l'étape 130. Cette matrice peut être obtenue par calcul, selon l'expression (17). Cependant, les inventeurs ont estimé qu'il est préférable d'établir la matrice de passage de façon expérimentale. Chaque ligne $M(c,n)$ de la matrice $M$ peut être considérée comme une fonction induisant une déformation de part et d'autre du terme diagonal $M(c, c)$, ce dernier étant tel que $M(c, c) = 1$

$$\frac{1}{\left(1 - e^{-att_{15}(c)}\right)}. \quad (25)$$

Ainsi, chaque terme de la ligne $M(c,n)$ peut être explicité selon l'expression :

$$M(c,n) = \frac{1}{\left(1 - e^{-att_{15}(c)}\right)} \times f\big(c, n, \alpha_1 \dots \alpha_p\big) (26),$$

où f est une fonction de déformation et $\alpha_1 \dots \alpha_p$ étant des paramètres de la fonction de déformation $f$.

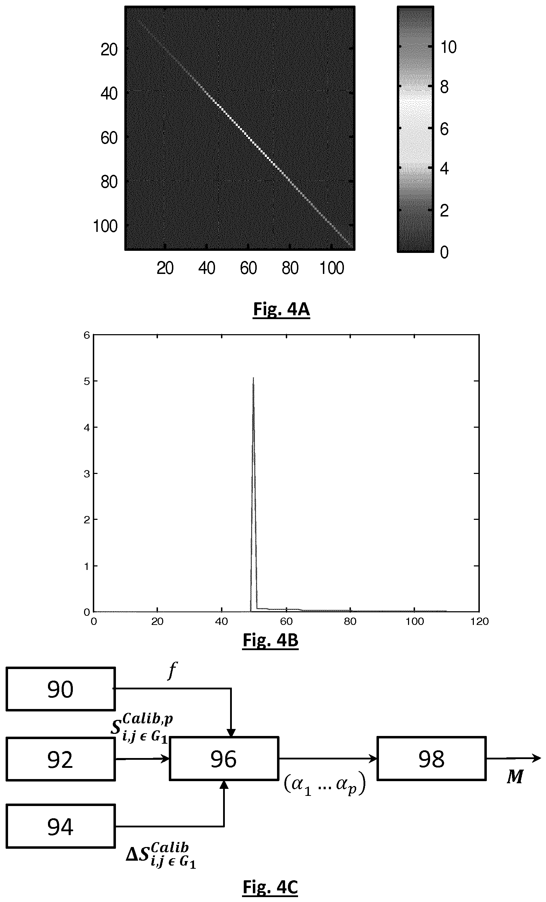

L'établissement de la matrice de passage nécessite la définition d'une expression analytique de la fonction de déformation $f$. Pour cela, on effectue des simulations selon différentes configurations de calibration, chaque configuration de calibration $Calib$ correspondant à un matériau de nature et d'épaisseur connue. Ces configurations constituent une base de calibrage. Pour chaque configuration de calibration $Calib$, on effectue les étapes suivantes, décrites en relation avec la figure 4C, en considérant une pluralité de pixels du détecteur appartenant au premier groupe $G_1$. :

- Etape 90 sélection d'une forme analytique de la fonction f, paramétrée par un ensemble de paramètres ($\alpha_1 \dots \alpha_p$) ;
- Etape 92 pour chaque configuration de calibration $Calib$, détermination, par simulation, d'un spectre du rayonnement primaire $S_{i,j \in G_1}^{Calib,p}$ ; ;
- Etape 94 : pour chaque configuration, détermination, par simulation ou mesure, d'un spectre du rayonnement atteignant le détecteur avec et sans masque, et calcul d'un spectre de comparaison $\Delta S_{i,j \in G_1}^{Calib}$, représentant une différence entre ces deux spectres ;
- Etape 96 : En utilisant les spectres $S_{i,j \in G_1}^{Calib,p}$ correspondant aux différentes configurations de calibration, détermination de l'ensemble de paramètres ($\alpha_1 \dots \alpha_p$) selon l'expression :

$$(\alpha_1 \ldots \alpha_p) = \underset{(\alpha_1 \ldots \alpha_p)}{\mathrm{argmin}} \, \psi \left( S_{i,j \, \epsilon \, G_1}^{Calib,p} ; \, \widehat{S}_{i,j \, \epsilon \, G_1}^{Calib,p} \right) \, (27)$$

où :

- $\widehat{S}_{i,j \, \epsilon \, G_1}^{Calib,p}$ est une estimation du spectre primaire $S_{i,j \, \epsilon \, G_1}^{Calib,p}$ obtenue en mettant en oeuvre la matrice de passage **M**, paramétrée par les paramètres $(\alpha_1 \ldots \alpha_p)$ selon l'expression : $\widehat{S}_{i,j \, \epsilon \, G_1}^{Calib,p} = M \times \Delta S_{i,j \, \epsilon \, G_1}^{Calib}$ ;

et $\psi$ est une fonction représentative d'une erreur, entre l'ensemble des spectres $S_{i,j \, \epsilon \, G_1}^{Calib,p}$ et leurs estimations $\widehat{S}_{i,j \, \epsilon \, G_1}^{Calib,p}$ respectives obtenues à l'aide de la matrice de passage **M**, cette dernière étant paramétrée par les paramètres $(\alpha_1 \ldots \alpha_p)$. Il peut s'agir d'une fonction de type erreur quadratique.

- Etape 98 : obtention de la matrice **M** en utilisant la fonction *f* dont les paramètres ont été déterminés lors de l'étape 96, et en utilisant l'expression (26).

Autres modes de réalisation

Selon un mode de réalisation, détaillé en lien avec les figures 5A et 5B, la position du détecteur et/ou de la source vis-à-vis de l'objet évolue entre deux mesures consécutives. Aussi, à chaque mesure correspond une configuration *C*, chaque configuration étant caractérisée par une position relative du détecteur 20 et/ou de la source 11 par rapport à l'objet 10, comme précédemment défini. Chaque configuration est caractérisée par un paramètre $P_C$, ce dernier représentant la position du détecteur et/ou de la source par rapport à l'objet. Ce paramètre peut être un scalaire ou un vecteur. Sur les exemples représentés sur les figures 5A et 5B, la source est fixe par rapport au détecteur et l'ensemble constitué par la source et le détecteur tourne autour de l'objet, selon un axe Y perpendiculaire au plan XZ, selon un angle $\theta$ par rapport à une position initiale. Cet angle constitue le paramètre de chaque configuration de mesure $P_C$.

Dans une première configuration *C*, représentée sur la figure 5A, $\theta = 0$. Le pixel $20_l$ est masqué par un élément absorbant $15_x$ du masque 15. Dans une deuxième configuration *C'*, représentée sur la figure 5B, $\theta = \theta > 0$. Le pixel $20_l$ n'est pas masqué par le masque 15. Dans un tel mode de réalisation, pour le pixel *l*, le premier spectre $S_l^{1,C}$ est obtenu selon la configuration *C* représentée sur la figure 5A, tandis que le deuxième spectre $S_l^{2,C}$ correspond au premier spectre $S_l^{1,C'}$ obtenu selon la configuration *C'*. Autrement dit, $S_l^{2,C} = S_l^{1,C'}$. Ce mode de réalisation permet de conserver un masque fixe entre la source et l'objet, le deuxième spectre $S_{i,j}^{2,C}$ d'un pixel $20_{i,j}$ étant obtenu lors de la mesure du premier spectre $S_{i,j}^{1,C'}$ la configuration *C'*. Lors de la première configuration *C*, le pixel $20_{i,j}$ appartient au premier groupe $G_{1,C}$, tandis que lors de la deuxième configuration *C'*, ce pixel n'appartient pas au premier groupe $G_{1,C'}$.

De préférence, ces deux configurations sont suffisamment comparables pour que le spectre mesuré par le pixel $20_l$, dans la deuxième configuration, soit représentatif du rayonnement, sans masque, lors de la première configuration. Ainsi, de préférence, les paramètres *P* et *P'* respectivement associé aux configurations *C* et *C'* sont voisins. Par voisin, il est entendu que leur écart relatif n'excède pas un seuil prédéterminé, par exemple 10 ou 20%.

Selon un mode de réalisation, le procédé est mis en oeuvre selon une pluralité de configurations *C*, à chaque configuration étant associée une position du détecteur et de la source par rapport à l'objet, de façon à obtenir, dans chaque configuration, pour une pluralité de pixels, un spectre corrigé $S_{i,j}^{*,C}$. Les spectres corrigés $S_{i,j}^{*,C}$ associés à chaque configuration $S_{i,j}^{*,C}$ sont utilisés pour réaliser une reconstruction tomographique de l'objet 10. Chaque configuration peut être paramétrée par un paramètre $P^C = \theta^C$, représentant un angle formé par l'ensemble source-détecteur et l'objet.

La reconstruction vise notamment à reconstruire la forme des éléments 10q constituant l'objet, ainsi que leur coefficient d'atténuation linéaire $\mu_q$ ou tout autre coefficient exprimant une atténuation. Elle est réalisée en mettant en oeuvre des algorithmes d'inversion connus, sur la base des images spectrales $Im^C(E)$ correspondant à chaque énergie *E*, chaque image représentant une quantité de rayonnement à ladite d'énergie, cette quantité étant obtenue d'après les spectres corrigés $S_{i,j}^{*,C}$. L'utilisation de ces spectres corrigés permet d'améliorer significativement la qualité de la reconstruction tomographique, comme décrit dans les exemples qui suivent.

Préalablement à la reconstruction, on peut procéder à un regroupement de spectres dans des bandes spectrales $\Delta E$, typiquement de quelques dizaines de keV, afin de constituer des spectres $S_{i,j}^{*,C}(\Delta E)$ dans chacune de ces bandes spectrales, et d'obtenir des images spectrales $Im^C(\Delta E)$ correspondant à des bandes spectrales.

Exemples

On va à présent décrire des exemples de réalisations de l'invention, basés sur des simulations réalisées par code de calcul, selon une application en radiographie et une application en tomographie. Préalablement, on décrit un exemple d'établissement d'une matrice de passage **M.**

Comme décrit en lien avec la figure 4C, une matrice de passage est déterminée à l'aide d'une base de calibrage, comportant un ou plusieurs matériaux. Dans cet exemple, on considère 2 matériaux (eau et aluminium), les épaisseurs considérées étant :

- pour l'aluminium, entre 0 et 0.2 cm selon un pas de 0.1 cm ;
- pour l'eau, entre 0 et 20 cm, selon un pas de 0.5 cm.

La forme analytique de la fonction *f* est choisie de telle sorte que :

- pour n≥c, $$f(c, n, \alpha_1, \alpha_2) = 0.9e^{-\frac{(n-c)^2}{2\alpha_1^2}} + 0.1e^{-\alpha_2 n} \quad (30)$$

- pour n < c $$f(c, n, \alpha_1, \alpha_2) = e^{-\frac{(n-c)^2}{2\alpha_1^2}} \quad (31)$$

Les indices c et *n* sont respectivement associés à une ligne et à une colonne de la matrice de passage.

Par ailleurs, pour chaque configuration de calibration, on a modélisé le spectre primaire $S_{i,j \in G_1}^{Calib,p}$ atteignant les pixels du premier groupe du détecteur mis en oeuvre et on a effectué des mesures simulées du spectre avec et sans masque, de façon à constituer un spectre de comparaison $\Delta S_{i,j \in G_1}^{Calib}$. On a ensuite déterminé les valeurs $\alpha_1$ et $\alpha_2$ comme précédemment décrit. On aboutit à $\alpha_1 = 0.2$ et $0.04 \le \alpha_2 \le 0.18$. La figure 4A représente la matrice de passage M obtenu, tandis que la figure 4B représente une colonne de cette matrice de coordonnée n= 45.

On décrit à présent des simulations mettant en oeuvre le dispositif représenté sur les figures 1A et 1B. L'objet est un fantôme simulant le torse d'un être humain. Il comporte une matrice $10_0$, dont la section, dans un plan parallèle au plan XZ du faisceau d'irradiation, est ovale, les plus grandes dimensions selon les axes Z et X étant respectivement de 20 cm et de 40 cm. Cette matrice $10_0$ représente le corps et est constituée d'eau. Un premier élément $10_1$ et un deuxième élément $10_2$ disposés à l'intérieur de la matrice $10_0$, représentent les poumons. Leur section, dans le plan XZ, est ovale, les plus grandes dimensions selon les axes Z et X étant respectivement de 11 cm et de 15 cm. Ces éléments sont constitués d'air. Un troisième élément $10_3$ représente la colonne vertébrale. Sa section, dans le plan XZ, est en forme de disque de diamètre 3 cm Un quatrième élément $10_4$, de forme rectangulaire, représente une vertèbre. Le matériau le constituant est un os, modélisé selon une base de données de l'ICRU (International Commission on Radiation Units and Measurements). Enfin, la périphérie de la matrice $10_5$ est constituée par un os annulaire de section ovale, représentant les cotes. Son épaisseur est de 1 cm. Des inclusions sensiblement sphériques $10_7$, $10_8$ et $10_9$ de diamètre 2 cm sont disposées au niveau des premier et deuxième éléments, représentant les poumons. Ces inclusions sont constituées de PMMA (Polyméthacrylate de méthyle) et représentent des tumeurs cancéreuses.

La source d'irradiation 11 est un tube à rayons X avec une anode de tungstène, soumis à une tension de 110 kV. Le détecteur 20 comprend 640 (selon l'axe X) * 64 pixels (selon l'axe Y), chaque pixel comprenant une épaisseur de CdTe de 5 mm. La surface de chaque pixel, dans le plan XY selon lequel s'étend le détecteur, est de 1mm * 1mm. Le détecteur est résolu en énergie, et chaque pixel permet d'obtenir des spectres selon des canaux d'énergie de 1 keV. Le masque 15 utilisé est celui représenté sur la figure 2.

Les figures 6A, 6B et 6C représentent respectivement le rayonnement total sans le masque, le rayonnement primaire et le rayonnement primaire estimé en mettant en oeuvre l'invention, selon le procédé représenté sur la figure 3, sur l'ensemble des pixels du détecteur. Lors de la mise en oeuvre du procédé, les deuxièmes spectres $S_{i,j \in G_1}^2$ sont obtenus en simulant un masque, tel que représenté sur la figure 2, placé à une distance de 10cm de la source. La grandeur représentée, selon le code couleur, correspondant à chaque pixel $20_{i,j}$ est une valeur de l'atténuation $A_{i,j}$, telle que

$$A_{i,j} = -log\left(\frac{\sum_n n s'_{i,j}(n)}{\sum_n n s^0_{i,j}(n)}\right) \quad (32)$$

où :

- $S^0_{i,j}$ désigne un spectre mesuré par un pixel $20_{i,j}$ lorsque le détecteur est directement exposé à la source. Cela représente le spectre du rayonnement incident $I^0$.

- $S'_{i,j}$ désigne un spectre d'intérêt mesuré par un pixel $20_{i,j}$. Sur la figure 6A, $S'_{i,j}$ représente le spectre du rayonnement transmis par l'objet, sans le masque : $S'_{i,j} = S_{i,j}$. Sur la figure 6B, $S'_{i,j}$ représente le spectre primaire du rayonnement transmis par l'objet, sans le masque, l'objet étant disposé entre la source et le détecteur : $S'_{i,j} = S^p_{i,j}$. Sur la figure 6C, $S'_{i,j}$ représente le spectre corrigé selon l'invention, qui correspond à une estimation du spectre primaire de primaire de $S_{i,j} : S'_{i,j} = S^*_{i,j} = \hat{S}^p_{i,j}$.

Chaque grandeur $A_{i,j}$ est représentative d'une atténuation globale dans l'objet, le terme globale désignant le fait qu'elle est déterminée pour l'ensemble des canaux d'énergie du spectre. Cela permet une représentation de chaque spectre d'intérêt $S'_{i,j}$ par un scalaire, ce qui facilite les illustrations. Les figures 6A, 6B et 6C montrent que :

- l'estimation du spectre du rayonnement primaire $\hat{S}^p_{i,j}$, mettant en oeuvre l'invention (figure 6C) est cohérente avec le spectre du rayonnement primaire modélisé $S^p_{i,j}$ (figure 6B) ;
- Les spectres du rayonnement primaire sont plus contrastés (figures 6B et 6C) que le spectre du rayonnement total (figure 6A). La séparation du spectre du rayonnement primaire permet une meilleure séparation spatiale de zones présentant des atténuations différentes. Cela est particulièrement visible sur la partie centrale des images, correspondant aux éléments denses $10_3$ et $10_4$ (os). Le recours au spectre du rayonnement primaire permet d'obtenir une valeur de l'atténuation plus conforme à la réalité.

Ces résultats sont confirmés sur la figure 6D, représentant, un profil central de chacune de ces images selon une valeur même valeur de y = 32. Sur cette figure, les indices A, B et C se réfèrent respectivement aux figures 6A, 6B et 6C.

Les figures 7A, 7B, 7C et 7D représentent les spectres du rayonnement total $S_{i,j}$, du rayonnement primaire modélisé $S^p_{i,i}$ et du rayonnement primaire estimé $\hat{S}^p_{i,j}$ selon l'invention respectivement sur les pixels $20_{ij}$ du détecteur de coordonnées (i = 100, j = 15 ; i = 192, j = 38 ; i = 32, j = 55 ; i = 320, j = 32). Sur chacune de ces figures, les indices 1, 2 et 3 représentent respectivement le spectre du rayonnement total $S_{i,j}$, le spectre du rayonnement primaire modélisé $S^p_{i,j}$ ainsi que le spectre du rayonnement primaire $\hat{S}^p_{i,j}$ estimé selon l'invention. On remarque la cohérence, en chacun des points, entre les spectres du rayonnement primaire modélisés et estimés selon l'invention. Le pixel i = 320, j = 32 , représenté sur la figure 7D, est remarquable par le fait que la quasi-totalité du rayonnement détecté est un rayonnement diffusé, la contribution du rayonnement primaire étant négligeable. Ce pixel est placé en vis-à-vis des éléments osseux $10_3$ et $10_4$, fortement atténuants. Cela signifie que le rayonnement mesuré par ce pixel est essentiellement constitué de rayonnement diffusé provenant de l'objet. On mesure ici l'intérêt et l'efficacité de l'invention.

L'efficacité d'une méthode de correction du spectre diffusé est fréquemment mesurée par un indicateur, noté SPR, acronyme de Scatter-to-Primary-Ratio, signifiant ratio diffusé sur primaire.

Une première expression de cet indicateur, dite expression intégrale, est telle que :

$$SPR = \frac{\sum_n \sum_{i,j} n S_{i,j}(n)}{\sum_n \sum_{i,j} n S_{i,j}^p(n)} \times 100 \quad (33)$$

Le terme « intégrale » désigne le fait que l'indicateur est réalisé pour l'ensemble des pixels et des canaux d'énergie.

Cet indicateur peut être pixelisé, c'est-à-dire être associé à chaque pixel $20_{i,j}$ du détecteur, auquel cas il est explicité par une deuxième expression, dite expression spatiale :

$$SPR(i,j) = \frac{\sum_n n S_{i,j}(n)}{\sum_n n S_{i,j}^p(n)} \times 100 \quad (34)$$

Il peut également être exprimé en fonction de l'énergie, sur l'ensemble des pixels du détecteur auquel cas il est explicité selon une troisième expression, dite expression spectrale :

$$SPR(n) = \frac{\sum_{i,j} n S_{i,j}(n)}{\sum_{i,j} n S_{i,j}^p(n)} \times 100 \quad (35)$$

Quelle que soit son expression, intégrale, spatiale ou spectrale, plus le coefficient SPR est faible, plus la part de diffusé respectivement sur l'ensemble du détecteur, dans le pixel $20_{i,j}$, ou dans la plage d'énergie $n$, est faible.

Il est également possible de quantifier l'efficacité de l'estimation du spectre du rayonnement primaire par un indicateur différentiel normalisé *Ind_diff,* traduisant l'efficacité de l'estimation pour chaque pixel $20_{i,j}$ du détecteur. A l'instar du coefficient SPR précédemment décrit, cet indicateur *Ind_diff* peut s'exprimer de la façon intégrale, spatiale ou spectrale, respectivement selon les expressions :

$$Ind\_diff = \frac{\sum_n \sum_{i,j} n\left(\left|\hat{S}_{i,j}^p(n) - S_{i,j}^p(n)\right|\right)}{\sum_n \sum_{i,j} n S_{i,j}^p(n)} \times 100 \quad (36)$$

$$Ind\_diff(i,j) = \frac{\sum_n n\left(\left|\hat{S}_{i,j}^p(n) - S_{i,j}^p(n)\right|\right)}{\sum_n n S_{i,j}^p(n)} \times 100 \quad (37)$$

$$Ind\_diff(n) = \frac{\sum_{i,j} n\left(\left|\hat{S}_{i,j}^p(n) - S_{i,j}^p(n)\right|\right)}{\sum_{i,j} n S_{i,j}^p(n)} \times 100 \quad (38)$$

Quelle que soit son expression, intégrale, spatiale ou spectrale, plus le coefficient *Ind_diff* est faible, meilleure est l'estimation.

Le tableau 1 représente, sur l'ensemble des pixels du détecteur, les différents indicateurs décrits ci-dessus :

Tableau 1

|  | Integral | Spatial | | | Spectral | | |
|---|---|---|---|---|---|---|---|
|  |  | Min | Max | Moyen | Min | Max | Moyen |
| SPR | 19.5% | 7.10% | $1.06 \times 10^3$% | 86.6% | 11.4% | 31.9% | 21.6% |
| *Ind_diff* | 4.31% | 2.20% | 88.2% | 10% | 2.64% | 8.36% | 4.75% |

Sur l'image considérée, le ratio intégral signal sur primaire est voisin de 20%. On constate que l'indicateur *Ind_diff* reste en moyenne très faible, ce qui atteste de la bonne qualité de l'estimation.

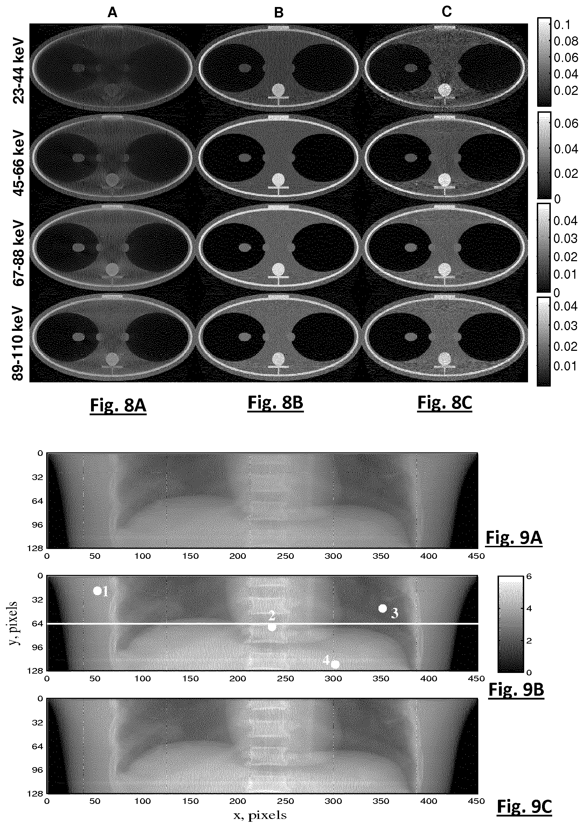

Une reconstruction tomographique de l'objet représenté sur la figure 1 a été réalisée, en simulant une rotation du dispositif de mesure (source, masque, détecteur) autour de l'objet, l'axe de rotation étant parallèle à l'axe Z, et en réalisant une acquisition par pas angulaire de 1 degré. Avant la reconstruction, les canaux des spectres sont regroupés en 4 bandes spectrales de largeur 21 keV entre 23 keV et 110 keV . Ces bandes spectrales sont 23 keV - 44 keV ; 45 keV - 66 keV ; 67 keV - 88 keV ; 89 keV - 110 keV. Les figures 8A, 8B et 8C représentent une coupe de l'atténuation de l'objet reconstruit, respectivement sur la base :

- du spectre du rayonnement total $S_{i,j}$ ;

- du spectre du rayonnement primaire simulé $S_{i,j}^{p}$ ;

- du spectre du rayonnement primaire estimé selon l'invention $\widehat{S_{i,j}^{p}}$, après avoir simulé, à chaque pas angulaire, une mesure avec et sans masque.

Chaque spectre a été modélisé selon une résolution spectrale de 1 keV. Puis on a procédé à un regroupement de canaux, de façon à obtenir les 4 bandes spectrales précédemment décrites. La reconstruction selon chaque bande spectrale est représentée sur les figures 8A, 8B et 8C.

On remarque une bonne cohérence entre les reconstructions réalisées sur la base du rayonnement primaire simulé et sur la base du rayonnement primaire estimé selon l'invention.

On constate également que la prise en compte du rayonnement primaire a d'autant plus d'effet que l'énergie est faible.

Des essais expérimentaux ont été réalisés en utilisant un fantôme anthropomorphique représentant le thorax d'une personne (traduire *Anthropomorphic thorax phantom).* Les conditions expérimentales sont :

- source de radiation : X-ray tube YXLON Y.TU 160-D06 - tension de polarisation 110 kV ;
- détecteur : détecteur CdTe MutliX ME 100 128 x 1 pixels résolu en énergie- pitch 0.8 mm - épaisseur 3 mm. Ce détecteur linéaire a été translaté pour obtenir des images de 128 x 451 pixels ;
- masque constitué d'un array de 11 x 21 cylindres d'aluminium, de diamètre 2 mm et de hauteur 2 mm, la distance entre deux cylindres adjacents étant de 5 mm.
- La matrice de passage **M** a été établie en utilisant des cylindres d'aluminium et de PMMA (Polymethyl méthacrylate) de diamètre 2 cm. L'épaisseur des cylindres d'aluminium varie entre 0 cm et 16 cm par incréments de 4 cm. L'épaisseur des cylindres de PMMA varie entre 0 cm et 3 cm par incréments de 1 cm.

Les figures 9A, 9B et 9C représentent respectivement une atténuation $A_{i,j}$, telle que décrite en lien avec l'équation (32), calculée dans une plage d'énergie 25 keV-110 keV, et calculée sur la base :

- d'un spectre du rayonnement transmis, mesuré par chaque pixel $20_{i,j}$
- d'un spectre du rayonnement transmis auquel on a soustrait une estimation d'un rayonnement diffusé. Cette estimation est réalisée en plaçant une bande atténuante, dite Beam Stop. Les pixels situés face à cette bande atténuante mesurent un spectre représentatif du spectre diffusé. Ce spectre est ensuite soustrait du spectre transmis, de façon à obtenir une estimation du spectre primaire pour chaque pixel . Il s'agit d'une méthode de correction de référence.
- d'un spectre du rayonnement primaire estimé selon l'invention, sur chaque pixel $20_{i,j}$ ;

On observe une bonne cohérence des figures 9B et 9C, ce qui atteste de la validité de la correction selon l'invention.

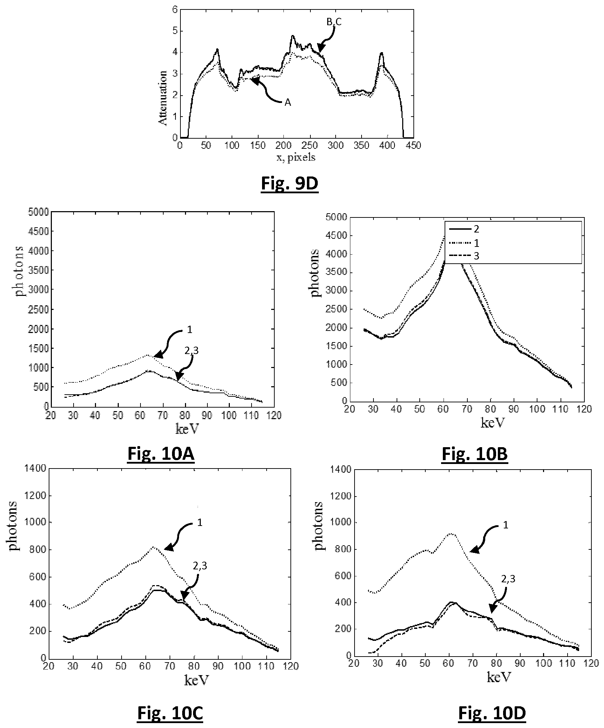

Sur le figure 9B, on a représenté une ligne horizontale. La figure 9D représente un profil de l'intensité sur chacune des figures 9A à 9C selon cette ligne. On observe la bonne cohérence entre les profils issus des figures 9B et 9C. Sur cette figure, les indices A, B et C se réfèrent respectivement aux figures 9A, 9B et 9C.

Les figures 10A, 10B, 10C et 10D représentent chacune les spectres du rayonnement transmis, corrigé selon une méthode de référence et corrigé selon l'invention, respectivement sur chaque point 1,2, 3 et 4 représenté sur la figure 9B. Sur chacune de ces figures, les indices 1, 2 et 3 représentent respectivement le spectre du rayonnement total $S_{i,j}$, le spectre du rayonnement primaire $S_{i,j}^{p}$ obtenu selon la méthode de référence, ainsi que le spectre du rayonnement primaire $\widehat{S}_{i,j}^{p}$ estimé selon l'invention.

L'invention pourra s'appliquer dans des procédés d'imagerie spectrale mettant en oeuvre des rayonnements ionisants, en particulier des rayonnements X ou gamma, pour des applications médicales ou plus généralement, dans le contrôle non destructif d'objets, visant à investiguer la structure interne dudit objet. L'objet peut être, par exemple, un bagage, un produit industriel, un élément de structure d'une installation, par exemple une tuyauterie, un déchet nucléaire...

L'invention permet une estimation de la composante primaire d'un rayonnement, limitant ainsi l'influence du rayonnement diffusé. On améliore alors la qualité de l'image obtenue, et en particulier la résolution spatiale. Il en résulte des résultats plus précis, et plus conformes à l'objet examiné.

La mise en oeuvre du procédé est simple et peut s'adapter à des dispositifs existants. De plus, la matrice de passage peut être préalablement établie, ce qui permet une mise en oeuvre du procédé rapide, ne nécessitant pas de temps de calcul élevé. Le procédé est donc adapté à une mise en oeuvre à une cadence industrielle.

L'invention pourra s'appliquer dans des procédés d'imagerie spectrale mettant en oeuvre des rayonnements ionisants, en particulier des rayonnements X ou gamma, pour des applications médicales ou plus généralement, dans le contrôle non destructif d'objets, visant à investiguer la structure interne dudit objet. L'objet peut être, par exemple, un bagage, un produit industriel, un élément de structure d'une installation, par exemple une tuyauterie, un déchet nucléaire...

L'invention permet une estimation de la composante primaire d'un rayonnement, limitant ainsi l'influence du rayonnement diffusé. On améliore alors la qualité de l'image obtenue, et en particulier la résolution spatiale. Il en résulte des résultats plus précis, et plus conformes à l'objet examiné.

La mise en oeuvre du procédé est simple et peut s'adapter à des dispositifs existants. De plus, la matrice de passage peut être préalablement établie, ce qui permet une mise en oeuvre du procédé rapide, ne nécessitant pas de temps de calcul élevé. Le procédé est donc adapté à une mise en oeuvre à une cadence industrielle.

## Revendications

1. Procédé de correction d'un spectre d'un rayonnement électromagnétique ionisant transmis par un objet (10),

   - l'objet étant disposé entre une source d'irradiation (11) et un détecteur (20), la source d'irradiation étant apte à émettre un rayonnement électromagnétique ionisant, dit rayonnement incident $(I^0)$, vers ledit objet ;
   - le détecteur (20) comportant des pixels $(20_{i,j})$, chaque pixel étant configuré pour détecter un rayonnement transmis par l'objet (10) vers le détecteur, et à en acquérir un spectre $(\mathbf{S}_{i,j})$, le rayonnement transmis comportant un rayonnement de diffusion $(I^{diff})$, causé par la diffusion du rayonnement incident dans l'objet (10), et un rayonnement primaire $(I^p)$ ;

   le procédé comportant les étapes suivantes :

   a) interposition d'un masque (15) entre la source (11) et l'objet (10), et acquisition, par plusieurs pixel $(20_{i,j})$, d'un premier spectre $(S_{i,j}^1)$ d'un premier rayonnement $(I^1)$ transmis par l'objet, ledit masque comportant des éléments absorbants $(15_x)$, aptes à absorber une partie dudit rayonnement incident $(I^0)$, et dont une projection sur le détecteur définit un premier groupe de pixels $(G_1)$;

   b) obtention, pour chaque pixel du premier groupe de pixels $(G_1)$, d'un deuxième spectre $(S_{i,j \in G_1}^2)$ d'un deuxième rayonnement $(I^2)$ transmis par l'objet sur le détecteur, en l'absence dudit masque ;

   c) comparaison, pour chaque pixel dudit premier groupe de pixels $(G_1)$, du premier spectre $(S_{i,j \in G_1}^1)$ et du deuxième spectre $(S_{i,j \in G_1}^2)$, de façon à obtenir un spectre de comparaison $(\Delta \mathbf{S}_{i,j \in G_1})$;

   d) pour chaque pixel du premier groupe de pixels $(G_1)$, application d'une matrice de passage ($\boldsymbol{M}$), préalablement établie, audit spectre de comparaison $(\Delta \mathbf{S}_{i,j \in G_1})$, pour obtenir une estimation d'un spectre dit primaire $(\widehat{S}_{i,j \in G_1}^{2\ p})$ représentant le rayonnement primaire transmis par l'objet audit pixel, et estimation d'un spectre de diffusion $(\tilde{S}_{i,j \in G_1}^{diff})$, représentatif du rayonnement de diffusion transmis par l'objet ;

   e) pour tout ou partie des pixels $(20_{i,j})$ du détecteur (20), à partir de chaque spectre de diffusion $(\tilde{S}_{i,j \in G_1}^{diff})$ estimé lors de l'étape d), correction du deuxième spectre $(S_{i,j}^2)$ ou du premier spectre $(S_{i,j}^1)$, de façon à obtenir un spectre corrigé $(S_{i,j}^*)$.

2. Procédé selon la revendication 1, dans lequel l'étape e) comporte, préalablement à ladite correction, une estimation d'un spectre de diffusion $(\tilde{S}_{i,j}^{diff})$ pour l'ensemble des pixels du détecteur.

3. Procédé selon la revendication 1 ou la revendication 2, dans lequel l'étape e) comporte, pour chaque pixel, une soustraction dudit spectre de diffusion estimé $(\tilde{S}_{i,j}^{diff})$ audit deuxième spectre $(S_{i,j}^2)$ ou audit premier spectre $(S_{i,j}^1)$.

4. Procédé selon l'une quelconque des revendications précédentes, dans lequel, lors de l'étape b), pour chaque pixel du premier groupe ($G_1$), le deuxième spectre $(S_{i,j \in G_1}^2)$ est obtenu :

   - soit par une acquisition dudit deuxième spectre par ledit pixel ;
   - soit par une estimation sur la base d'au moins un premier spectre $(S_{i,j \notin G_1}^1)$ acquis par au moins un pixel n'appartenant pas audit premier groupe.

5. Procédé selon l'une quelconque des revendications précédentes, dans lequel :

   - l'étape a) est mise en oeuvre dans une pluralité de configurations (C), à chaque configuration étant associée une position du détecteur et de la source par rapport à l'objet, de façon à obtenir, à chaque configuration, et pour chaque pixel, un premier spectre $(S_{i,j}^{1,C})$, à chaque configuration étant également associé un premier groupe de pixel $(G_1^C)$ ;
   - l'étape b) comporte, pour tout ou partie des pixels du premier groupe $(G_1^C)$ associé à une configuration (C), une détermination d'un deuxième spectre $(S_{i,j}^{2,C})$ à partir d'un premier spectre $(S_{i,j}^{1,C'})$ obtenu selon une autre configuration (C').

6. Procédé selon la revendication 5, dans lequel à chaque configuration (C) est associé un paramètre ($P_C$), de telle sorte qu'au moins un deuxième spectre $(S_{i,j}^{2,C})$ selon une configuration (C) associée à un premier paramètre ($P_C$) est obtenu à partir d'un premier spectre $(S_{i,j}^{1,C'})$ obtenu selon une autre configuration (C') associée à un deuxième paramètre ($P_{C'}$), différent du premier paramètre.

7. Procédé selon la revendication 6, dans lequel ledit paramètre ($P_C$) est un angle d'inclinaison ($\theta_C$) de la source (11) et/ou du détecteur (20) par rapport à l'objet (10).

8. Procédé selon l'une quelconque des revendications précédentes, dans lequel lors de l'étape d), l'estimation dudit spectre primaire comprend le produit matriciel de ladite matrice de passage ($M$) par chaque spectre de comparaison ($\Delta S_{i,j \in G_1}$).

9. Procédé selon l'une quelconque des revendications précédentes, dans lequel chaque élément absorbant ($15_x$) est apte à absorber entre 5 % et 80 % du rayonnement auquel il est exposé.

10. Procédé selon l'une quelconque des revendications précédentes, dans lequel, le masque (15) s'étendant selon une surface, chaque élément absorbant est distant d'un autre élément absorbant d'une distance inférieure à 1 cm.

11. Procédé selon l'une quelconque des revendications précédentes, dans lequel les étapes a) à e) sont mise en oeuvre selon une pluralité de configurations (C), à chaque configuration étant associée une position du détecteur et de la source par rapport à l'objet, de façon à obtenir, dans chaque configuration, pour une pluralité de pixels, un spectre corrigé $(S_{i,j}^{*,C})$, les spectres corrigés de chaque configuration étant utilisés pour réaliser une reconstruction tomographique de l'objet (10).

12. Procédé selon l'une quelconque des revendications précédentes, dans lequel les étapes a) à e) sont complétées par les étapes suivantes :

f) sélection d'au moins une énergie ($E$) ou une plage d'énergie ($\Delta E$);

g) réalisation d'une image ($Im(\Delta E)$, $Im(E)$), dont chaque pixel comprend une donnée obtenue à partir d'un spectre corrigé $(S^*_{i,j})$, associé à un pixel ($20_{i,j}$) du détecteur, à ladite énergie ($E$) ou dans ladite plage d'énergie ($\Delta E$).

13. Procédé selon la revendication 12, dans lequel lors de l'étape g) chaque pixel de l'image comporte une information relative à une intégrale ou à une moyenne dudit spectre corrigé $(S^*_{i,j})$ dans ladite plage d'énergie ($\Delta E$).

14. Procédé selon l'une quelconque des revendications précédentes, dans lequel le masque (15) est interposé entre la source d'irradiation (11) et l'objet (10).

15. Procédé selon l'une quelconque des revendications précédentes, dans lequel la matrice de passage *(M)* est obtenue en réalisant une pluralité de mesures dites de calibration, chaque mesure de calibration étant effectuée en interposant un matériau de nature et d'épaisseur connue entre la source d'irradiation (11) et le détecteur (20).

16. Support d'enregistrement d'informations, comportant des instructions pour l'exécution des étapes c) à e) du procédé selon l'une quelconque des revendications 1 à 15, ces instructions étant aptes à être exécutées par un microprocesseur.

17. Dispositif pour la réalisation d'images d'un objet (10) comportant :

- une source d'irradiation (11), apte à émettre un rayonnement électromagnétique ionisant, dit rayonnement incident ($I^0$), vers ledit objet (10);
- un détecteur (20) comportant des pixels ($20_{i,j}$), chaque pixel étant configuré pour détecter un rayonnement transmis par l'objet (10) vers le détecteur, et à en acquérir un spectre ($S_{i,j}$) ;
- un masque (15), apte à être interposé entre la source (11) et l'objet (10), ledit masque comportant des éléments absorbants ($15_x$), aptes à absorber une partie dudit rayonnement incident ($I^0$), et dont une projection sur le détecteur définit un premier groupe de pixels ($G_1$);
- un processeur (21), configuré pour recevoir des spectres acquis par chaque pixel, et à mettre en oeuvre le procédé objet des étapes c) à e) de l'une quelconque des revendications 1 à 15.

**Patentansprüche**

1. Verfahren zur Korrektur eines Spektrums einer von einem Objekt (10) übertragenen ionisierenden elektromagnetischen Strahlung,

- wobei das Objekt zwischen einer Strahlungsquelle (11) und einem Detektor (20) angeordnet ist, wobei die Strahlungsquelle geeignet ist, eine ionisierende elektromagnetische Strahlung, eintreffende Strahlung ($I^0$) genannt, zu dem Objekt zu senden;
- wobei der Detektor (20) Pixel ($20_{i,j}$) umfasst, wobei jedes Pixel dazu vorgesehen ist, eine von dem Objekt (10) zum Detektor übertragene Strahlung zu erkennen und davon ein Spektrum ($S_{i,j}$) zu erfassen, wobei die übertragene Strahlung eine Diffusionsstrahlung ($I^{diff}$), die durch die Diffusion der eintreffenden Strahlung in dem Objekt (10) verursacht wird, und eine primäre Strahlung ($I^p$) umfasst;

wobei das Verfahren die folgenden Schritte umfasst:

a) Zwischenfügung einer Maske (15) zwischen die Quelle (11) und das Objekt (10) und durch mehrere Pixel ($20_{i,j}$) Erfassung eines ersten Spektrums $(S^1_{i,j})$ einer ersten Strahlung ($I^1$), die von dem Objekt übertragen wird, wobei die Maske absorbierende Elemente ($15_x$) umfasst, die geeignet sind, einen Teil der eintreffenden Strahlung ($I^0$) zu absorbieren, und deren Projektion auf den Detektor eine erste Pixelgruppe ($G_1$) definiert;

b) für jedes Pixel der ersten Pixelgruppe ($G_1$) Erhalt eines zweiten Spektrums $(S^2_{i,j\in G_1})$ einer zweiten Strahlung ($I^2$), die von dem Objekt auf den Detektor übertragen wird, wobei die Maske nicht vorhanden ist;

c) für jedes Pixel der ersten Pixelgruppe ($G_1$) Vergleich des ersten Spektrums $\left(S^1_{i,j \in G_1}\right)$ und des zweiten Spektrums $\left(S^2_{i,j \in G_1}\right)$, um ein Vergleichsspektrum ($\Delta S_{i,j \in G_1}$) zu erhalten;

d) für jedes Pixel der ersten Pixelgruppe ($G_1$) Anwendung einer vorher erstellten Durchgangsmatrix (**M**) an dem Vergleichsspektrum ($\Delta S_{i,j \in G_1}$), um eine Schätzung eines so genannten primären Spektrums $\left(\widehat{S}^{2\,p}_{i,j \in G_1}\right)$, das die primäre Strahlung darstellt, die von dem Objekt auf das Pixel übertragen wird, und eine Schätzung eines Diffusionsspektrums $\left(\tilde{S}^{diff}_{i,j \in G_1}\right)$, das für die von dem Objekt übertragene Diffusionsstrahlung repräsentativ ist, zu erhalten;

e) für jedes oder einen Teil der Pixel ($20_{i,j}$) des Detektors (20) auf Basis jedes Diffusionsspektrums $\left(\tilde{S}^{diff}_{i,j \in G_1}\right)$, das in Schritt d) geschätzt wurde, Korrektur des zweiten Spektrums $\left(S^2_{i,j}\right)$ oder des ersten Spektrums $\left(S^1_{i,j}\right)$, um ein korrigiertes Spektrum $\left(S^*_{i,j}\right)$ zu erhalten.

2. Verfahren nach Anspruch 1, bei dem der Schritt e) vor der Korrektur eine Schätzung eines Diffusionsspektrums $\left(\tilde{S}^{diff}_{i,j \in G_1}\right)$ für die Gesamtheit der Pixel des Detektors umfasst.

3. Verfahren nach Anspruch 1 oder Anspruch 2, bei dem der Schritt e) für jedes Pixel eine Subtraktion des geschätzten Diffusionsspektrums $\left(\tilde{S}^{diff}_{i,j \in G_1}\right)$ vom zweiten Spektrum $\left(S^2_{i,j}\right)$ oder vom ersten Spektrum $\left(S^1_{i,j}\right)$ umfasst.

4. Verfahren nach einem der vorhergehenden Ansprüche, bei dem in Schritt b) für jedes Pixel der ersten Gruppe ($G_1$) das zweite Spektrum $\left(S^2_{i,j \in G_1}\right)$ erhalten wird:

   - entweder durch eine Erfassung des zweiten Spektrums durch das Pixel;

   - oder durch eine Schätzung auf Basis mindestens eines ersten Spektrums $\left(S^1_{i,j \notin G_1}\right)$, das von mindestens einem Pixel erfasst wird, das nicht der ersten Gruppe angehört.

5. Verfahren nach einem der vorhergehenden Ansprüche, bei dem:

   - der Schritt a) in einer Vielzahl von Konfigurationen ($C$) eingesetzt wird, wobei jeder Konfiguration eine Position des Detektors und der Quelle in Bezug zum Objekt zugeordnet ist, um in jeder Konfiguration und für jedes Pixel ein erstes Spektrum $\left(S^{1,C}_{i,j}\right)$ zu erhalten, wobei jeder Konfiguration auch eine erste Pixelgruppe $\left(G^C_1\right)$ zugeordnet ist;

   - der Schritt b) für alle oder einen Teil der Pixel der ersten Gruppe $\left(G^C_1\right)$, die einer Konfiguration ($C$) zugeordnet ist, eine Bestimmung eines zweiten Spektrums $\left(S^{2,C}_{i,j}\right)$ aus einem ersten Spektrum $\left(S^{1,C'}_{i,j}\right)$, das nach einer anderen Konfiguration ($C'$) erhalten wird, umfasst.

6. Verfahren nach Anspruch 5, bei dem jeder Konfiguration ($C$) ein Parameter ($P_C$) zugeordnet ist, so dass mindestens ein zweites Spektrum $\left(S^{2,C}_{i,j}\right)$ nach einer Konfiguration ($C$), die einem ersten Parameter ($P_C$) zugeordnet ist, aus einem ersten Spektrum $\left(S^{1,C'}_{i,j}\right)$ erhalten wird, das nach einer anderen Konfiguration ($C'$), die einem zweiten Parameter ($P_{C'}$), der sich vom ersten Parameter unterscheidet, zugeordnet ist, erhalten wird.

7. Verfahren nach Anspruch 6, bei dem der Parameter ($P_C$) ein Neigungswinkel ($\theta_C$) der Quelle (11) und/oder des Detektors (20) in Bezug zum Objekt (10) ist.

8. Verfahren nach einem der vorhergehenden Ansprüche, bei dem in Schritt d) die Schätzung des primären Spektrums das Matrixprodukt der Durchgangsmatrix (**M**) mal jedes Vergleichsspektrum ($\Delta S_{i,j \in G_1}$) umfasst.

9. Verfahren nach einem der vorhergehenden Ansprüche, bei dem jedes absorbierende Element ($15_x$) geeignet ist, zwischen 5 % und 80 % der Strahlung, der es ausgesetzt ist, zu absorbieren.

10. Verfahren nach einem der vorhergehenden Ansprüche, bei dem, wobei sich die Maske (15) entlang einer Oberfläche erstreckt, jedes absorbierende Element von einem weiteren absorbierenden Element um einen Abstand unter 1 cm entfernt ist.

11. Verfahren nach einem der vorhergehenden Ansprüche, bei dem die Schritte a) bis e) nach einer Vielzahl von Konfigurationen (C) eingesetzt werden, wobei jeder Konfiguration eine Position des Detektors und der Quelle in Bezug zum Objekt zugeordnet sind, um in jeder Konfiguration für eine Vielzahl von Pixel ein korrigiertes Spektrum ($S_{i,j}^{*,C}$) zu erhalten, wobei die korrigierten Spektren jeder Konfiguration verwendet werden, um eine tomografische Rekonstruktion des Objekts (10) durchzuführen.

12. Verfahren nach einem der vorhergehenden Ansprüche, bei dem die Schritte a) bis e) durch die folgenden Schritte ergänzt werden:

   f) Auswahl mindestens einer Energie ($E$) oder eines Energiebereichs ($\Delta E$);
   g) Herstellung eines Bildes ($Im(\Delta E)$, $Im(E)$), bei dem jedes Pixel ein Datum umfasst, das aus einem korrigierten Spektrum ($S_{i,j}^{*}$), das einem Pixel ($20_{i,j}$) des Detektors, der Energie ($E$) oder in dem Energiebereich ($\Delta E$) zugeordnet ist, erhalten wird.

13. Verfahren nach Anspruch 12, bei dem in Schritt g) jedes Pixel des Bildes eine Information zu einem Integral oder einem Durchschnitt des korrigierten Spektrums ($S_{i,j}^{*}$), in dem Energiebereich ($\Delta E$) umfasst.

14. Verfahren nach einem der vorhergehenden Ansprüche, bei dem die Maske (15) zwischen der Strahlungsquelle (11) und dem Objekt (10) zwischengefügt ist.

15. Verfahren nach einem der vorhergehenden Ansprüche, bei dem die Durchgangsmatrix (**M**) erhalten wird, indem eine Vielzahl von so genannten Kalibrierungsmessungen durchgeführt wird, wobei jede Kalibrierungsmessung durchgeführt wird, indem ein Material von bekannter Natur und Dicke zwischen der Strahlungsquelle (11) und dem Detektor (20) zwischengefügt wird.

16. Träger zur Aufzeichnung von Informationen, umfassend Befehle zur Ausführung der Schritte c) bis e) des Verfahrens nach einem der Ansprüche 1 bis 15, wobei diese Befehle geeignet sind, von einem Mikroprozessor ausgeführt zu werden.

17. Vorrichtung für die Herstellung von Bildern eines Objekts (10), umfassend:

   - eine Strahlungsquelle (11), die geeignet ist, eine ionisierende elektromagnetische Strahlung, eintreffende Strahlung ($I°$) genannt, zu dem Objekt (10) zu senden;
   - einen Detektor (20), umfassend Pixel ($20_{i,j}$), wobei jedes Pixel dazu vorgesehen ist, eine von dem Objekt (10) zum Detektor übertragene Strahlung zu erkennen und davon ein Spektrum ($S_{i,j}$) zu erfassen;
   - eine Maske (15), die geeignet ist, zwischen der Quelle (11) und dem Objekt (10) zwischengefügt zu werden, wobei die Maske absorbierende Elemente ($15_x$) umfasst, die geeignet sind, einen Teil der eintreffenden Strahlung ($I^0$) zu absorbieren, und von der eine Projektion auf den Detektor eine erste Pixelgruppe ($G_1$) definiert;
   - einen Prozessor (21), der dazu vorgesehen ist, von jedem Pixel erfasste Spektren zu empfangen und das Verfahren, das Gegenstand der Schritte c) bis e) eines der Ansprüche 1 bis 15 ist, einzusetzen.

**Claims**

1. Method for correcting a spectrum of an ionizing electromagnetic radiation transmitted by an object (10),

   - the object being arranged between an irradiation source (11) and a detector (20), the irradiation source being able to emit an ionizing electromagnetic radiation, called incident radiation $(I^0)$, towards said object;
   - the detector (20) comprising pixels $(20_{i,j})$, each pixel configured to detect a radiation transmitted by the object (10) towards the detector, and to acquire a spectrum $(S_{i,j})$ therefrom, the transmitted radiation comprising a scattering radiation $(I^{diff})$, caused by the scattering of the incident radiation in the object (10), and a primary radiation $(I^p)$ ;

   the method comprising the following steps:

   a) interposing a mask (15) between the source (11) and the object (10), and acquiring, by several pixels $(20_{i,j})$, a first spectrum $(S^1_{i,j})$ of a first radiation $(I^1)$ transmitted by the object, said mask comprising attenuating elements $(15_x)$, able to attenuate a part of said incident radiation $(I^0)$, and of which a projection on the detector defines a first group of pixels $(G_1)$;

   b) obtaining, for each pixel of the first group of pixels $(G_1)$, a second spectrum $(S^2_{i,j\in G_1})$ of a second radiation $(I^2)$ transmitted by the object to the detector, in the absence of said mask;

   c) comparing, for each pixel of said first group of pixels $(G_1)$, the first spectrum $(S^1_{i,j\in G_1})$ and the second spectrum $(S^2_{i,j\in G_1})$, so as to obtain a comparison spectrum $(\Delta S_{i,j\in G_1})$;

   d) for each pixel of the first group of pixels $(G_1)$, applying a transition matrix $(M)$, previously established, to said comparison spectrum $(\Delta S_{i,j\in G_1})$, to obtain an estimation of a so-called primary spectrum $(\widehat{S}^{2\,p}_{i,j\,\epsilon\,G_1})$ representing the primary radiation transmitted by the object to said pixel, and estimating a scattering spectrum $(\tilde{S}^{diff}_{i,j\,\epsilon\,G_1})$, representative of the scattering radiation transmitted by the object;

   e) for all or some of the pixels $(20_{i,j})$ of the detector (20), from each scattering spectrum $(\tilde{S}^{diff}_{i,j\,\epsilon\,G_1})$ estimated in the step d), correcting the second spectrum $(S^2_{i,j})$ or the first spectrum $(S^2_{i,j})$ so as to obtain a corrected spectrum $(S^*_{i,j})$.

2. Method according to Claim 1, in which the step e) comprises, prior to said correction, an estimation of a scattering spectrum $(\tilde{S}^{diff}_{i,j})$ for all of the pixels of the detector.

3. Method according to Claim 1 or Claim 2, in which the step e) comprises, for each pixel, a subtraction of said estimated scattering spectrum $(\tilde{S}^{diff}_{i,j})$ from said second spectrum $(S^2_{i,j})$ or said first spectrum $(S^1_{i,j})$.

4. Method according to any one of the preceding claims, in which, in the step b), for each pixel of the first group $(G_1)$, the second spectrum $(S^2_{i,j\in G_1})$ is obtained:

   - either by an acquisition of said second spectrum by said pixel;
   - or by an estimation on the basis of at least one first spectrum $(S^1_{i,j\notin G_1})$ acquired by at least one pixel not belonging to said first group.

5. Method according to any one of the preceding claims, in which:

24

- the step a) is implemented in a plurality of configurations ($C$), each configuration having associated with it a position of the detector and of the source relative to the object, so as to obtain, in each configuration and for each pixel, a first spectrum $(S_{i,j}^{1,C})$, each configuration also having associated with it a first group of pixels $(G_1^C)$;

- the step b) comprises, for all or some of the pixels of the first group $(G_1^C)$ associated with a configuration ($C$), a determination of a second spectrum $(S_{i,j}^{2,C})$ from a first spectrum $(S_{i,j}^{1,C'})$ obtained according to another configuration ($C'$).

6. Method according to Claim 5, in which each configuration ($C$) has associated with it a parameter ($P_C$), such that at least one second spectrum $(S_{i,j}^{2,C})$ according to a configuration ($C$) associated with a first parameter ($P_C$) is obtained from a first spectrum $(S_{i,j}^{1,C'})$ obtained according to another configuration ($C'$), the latter being associated with a second parameter ($P_{C'}$), different from the first parameter.

7. Method according to Claim 6, in which said parameter ($P_C$) is an angle of inclination ($\theta_C$)of the source (11) and/or of the detector (20) relative to the object (10).

8. Method according to any one of the preceding claims, in which, in the step d), the estimation of said primary spectrum comprises the matrix product of said transition matrix ($M$) by each comparison spectrum ($\Delta S_{i,j\in G_1}$).

9. Method according to any one of the preceding claims, in which each attenuating element ($15_x$) is able to attenuate between 5% and 80% of the radiation to which it is exposed.

10. Method according to any one of the preceding claims, in which, the mask (15) extending along a surface, each attenuating element is distant from another attenuating element by a distance less than 1 cm.

11. Method according to any one of the preceding claims, in which the steps a) to e) are implemented according to a plurality of configurations ($C$), each configuration having associated with it a position of the detector and of the source relative to the object, so as to obtain, in each configuration, for a plurality of pixels, a corrected spectrum $(S_{i,j}^{*,C})$, the corrected spectra of each configuration being used to produce a tomographic reconstruction of the object (10).

12. Method according to any one of the preceding claims, in which the steps a) to e) are complemented by the following steps:

f) selecting at least one energy ($E$) or an energy range ($\Delta E$);
g) producing an image ($Im(\Delta E)$, $Im(E)$), of which each pixel comprises a datum obtained from a corrected spectrum $(S_{i,j}^{*})$, associated with a pixel ($20_{i,j}$) of the detector, with said energy ($E$) or in said energy range ($\Delta E$).

13. Method according to Claim 12, in which, in the step g), each pixel of the image comprises an information item relating to an integral or to a mean of said corrected spectrum $(S_{i,j}^{*})$ in said energy range ($\Delta E$).

14. Method according to any one of the preceding claims, in which the mask (15) is interposed between the irradiation source (11) and the object (10).

15. Method according to any one of the preceding claims, in which the transition matrix ($M$) is obtained by performing a plurality of so-called calibration measurements, each calibration measurement being performed by interposing a material of known nature and thickness between the irradiation source (11) and the detector (20).

16. Information storage medium, comprising instructions for the execution of the steps c) to e) of the method according to any one of Claims 1 to 15, these instructions being able to be executed by a microprocessor.

**17.** Device for producing images of an object (10) comprising:

- an irradiation source (11), able to emit an ionizing electromagnetic radiation, called incident radiation $(I^0)$, towards said object (10);
- a detector (20) comprising pixels $(20_{i,j})$, each pixel being configured to detect a radiation transmitted by the object (10) towards the detector, and to acquire a spectrum $(\boldsymbol{S}_{i,j})$ therefrom;
- a mask (15), able to be interposed between the source (11) and the object (10), said mask comprising attenuating elements $(15_x)$, able to attenuate a part of said incident radiation $(I^0)$, and of which a projection onto the detector defines a first group of pixels $(G_1)$;
- a processor (21), configured to receive spectra acquired by each pixel, and to implement the method that is the subject of the steps c) to e) of any one of Claims 1 to 15.

**Fig. 1A**

**Fig. 1B**

**Fig. 1C**

**Fig. 2A**

**Fig. 2B**

**Fig. 2C**

**Fig. 3**

**Fig. 4A**

**Fig. 4B**

**Fig. 4C**

**Fig. 5A**

**Fig. 5B**

**Fig. 6A**

**Fig. 6B**

**Fig. 6C**

**Fig. 6D**

**Fig. 7A**

**Fig. 7B**

**Fig. 7C**

**Fig. 7D**

**Fig. 8A**  **Fig. 8B**  **Fig. 8C**

**Fig. 9A**

**Fig. 9B**

**Fig. 9C**

**Fig. 9D**

**Fig. 10A**

**Fig. 10B**

**Fig. 10C**

**Fig. 10D**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20120140891 A

**Littérature non-brevet citée dans la description**

- **SOSSIN A.** Fast scattering simulation tool for multi-energy x-ray imaging. *Nuclear Instruments and Methods in Physics Research,* 2015, vol. A 802, 60-66
- **ZHU L.** Scatter Correction Method for X-ray CT Using Primary Modulation : Theory and Preliminary Results. *IEEE Transactions on Médical Imaging,* Décembre 2006, vol. 25 (12
- **NING R.** X-ray Scatter Correction Algorithm for Cone Beam CT-Imaging. *Med.Phys.,* Mai 2004, vol. 31 (5
- **YANG K.** A breast-specific, negligible-dose scatter correction technique for dedicated cone-beam breast CT : a physics-based approach to improve Hounsfield Unit accuracy. *Phys.Med.Biol.,* 2014, vol. 59, 6487-6505